# FASCICULE DE BREVET EUROPEEN

(11) **EP 3 116 599 B1**
(45) Date de publication et mention de la délivrance du brevet: **23.12.2020**
(21) Numéro de dépôt: 15714589.7
(22) Date de dépôt: 09.03.2015
(51) Int. Cl.: A61Q 19/00, A61Q 19/08, A61K 8/97, A61K 36/51, A61K 8/60, A61K 8/9789, A61P 17/00

(54) **UTILISATIONS COSMÉTIQUES DE LA SWERTIAMARINE**
KOSMETISCHE VERWENDUNG VON SWERTIA-MARINE
COSMETIC USE OF SWERTIA-MARINE

(30) Priorité: 10.03.2014 FR 1451920
(43) Date de publication de la demande: 18.01.2017
(73) Titulaire: Lucas Meyer Cosmetics, F-92080 Paris La Défense (FR)
(72) Inventeur: BEZIVIN, Carine, F-91370 Verrieres le Buisson (FR)
(74) Mandataire: Cabinet Becker et Associés
(86) Numéro de dépôt international: PCT/FR2015/050569
(87) Numéro de publication internationale: WO 2015/136198

(56) Documents cités:
- EP-A1- 1 023 889
- JP-A- 2005 002 056
- JP-A- 2008 001 602
- KR-A- 20110 072 997
- DATABASE GNPD [Online] MINTEL; janvier 2014 (2014-01), Dr Ci:Labo: "Sleeping Mask", XP002732077, Database accession no. 2298297
- DATABASE GNPD [Online] MINTEL; janvier 2014 (2014-01), Emami: "Lalima Blood and Skin Purifier", XP002732078, Database accession no. 2281373

## Description

### Domaine de l'invention

La présente invention se rapporte au domaine cosmétique, en particulier aux agents cosmétiques capables de stimuler la formation ou la régénération de la peau.

### Contexte de l'invention

La peau est la première barrière protégeant l'organisme des agressions extérieures. Cet organe est composé par plusieurs couches de tissu. On distingue l'épiderme qui est la partie la plus externe de la peau, le derme, un tissu conjonctif constitué de fibroblastes et d'une matrice extracellulaire, qui assure les fonctions de cohésion et de nutrition de la peau, et l'hypoderme constitué d'adipocytes.

L'épiderme est constitué par plusieurs strates cellulaires de kératinocytes. On distingue, entre autres, la couche germinative de l'épiderme, appelée couche basale, contenant, notamment, les cellules souches cutanées, la couche épineuse, *Stratum spinosum,* constituée de plusieurs couches de cellules polygonales, la couche granuleuse, *Stratum granulosum,* comprenant une à trois couches de cellules aplaties contenant des inclusions cytoplasmiques, les grains de kératohyaline, et enfin, le couche cornée, *Stratum corneum* qui est composée de cellules anuclées et riches en kératine appelées cornéocytes qui correspondent au stade terminal de différenciation des kératinocytes.

Les cellules les plus externes de la couche cornée sont continuellement éliminées et remplacées par les cellules d'une couche inférieure, selon un processus appelé desquamation. La régénération cellulaire de la couche cornée est basée sur un processus de maturation cellulaire dans lequel les cellules de la couche basale de l'épiderme se différencient et migrent progressivement à travers les différentes strates de l'épiderme jusqu'à arriver à la couche cornée sous la forme de cornéocytes.

Le vieillissement cutané, qu'il résulte d'un phénomène normal de sénescence ou qu'il soit accentué par un facteur extérieur tel que l'exposition aux radiations UV, implique des dysfonctionnements de la différenciation et/ou du renouvellement cellulaire se traduisant par une atrophie de l'ensemble des assises de la peau.

D'un point de vue histologique, on observe, entre autres, une diminution de la qualité du derme, en particulier une perte de consistance de la matrice extracellulaire, et une diminution de l'épaisseur de l'épiderme.

D'un point de vue esthétique, ces altérations se traduisent par une modification de l'aspect de la peau et de ses propriétés mécaniques : la peau est moins lisse, voire rugueuse, et peut devenir déshydratée voire sèche. Son microrelief est plus marqué, et peut présenter des ridules, pouvant conduire avec le temps à la formation de rides profondes. La peau peut présenter également une perte d'élasticité et de fermeté, et un teint moins lumineux.

La peau peut présenter d'autres altérations de son aspect visuel, en particulier des vergetures ou des rougeurs.

Il existe sur le marché de nombreux produits cosmétiques destinés à prévenir ou à atténuer les rides ou à atténuer les rougeurs ou les vergetures.

L'art antérieur décrit des compositions comprenant des extraits de Swertia. On trouve notamment sur la base Mintel une crème de nuit comprenant 11 acides aminés, 12 huiles essentielles, un extrait de *Chondrus crispus,* et trois extraits de plantes orientales, dont un extrait *de Swertia japonica* (Base Mintel, record ID : 2298297).

La demande de brevet KR20110072997 décrit une composition cosmétique ou pharmaceutique comprenant de la swertiamarine en tant qu'inhibiteur de trypsine pour soulager les démangeaisons chez les sujets souffrant de dermatite atopique.

Néanmoins, il reste, à l'heure actuelle, un besoin pour de nouveaux actifs pour prévenir ou traiter des altérations de la peau, notamment résultant du vieillissement cutané.

### Résumé de l'invention

Un premier objet selon l'invention est l'utilisation cosmétique de la swertiamarine ou d'un extrait végétal enrichi en swertiamarine pour stimuler la formation ou la régénération de l'épiderme et/ou pour stimuler le métabolisme du derme selon la revendication 1. Ladite swertiamarine ou ledit extrait végétal peuvent être utilisés à titre d'agent anti-âge, d'agent antiride, d'agent unificateur du teint, d'agent anti-rougeur ou d'agent pour lisser la peau.

L'extrait végétal enrichi en swertiamarine est, de préférence, un extrait obtenu à partir d'une espèce de *Swertia,* telle que *Swertia chirata* ou *Swertia milensis,* et qui comprend au moins 90% en poids de swertiamarine. La swertiamarine ou l'extrait végétal est présent, à titre d'agent actif, dans une composition, de préférence cosmétique, destinée à être administrée par voie topique.

Ladite composition est de préférence destinée à prévenir ou traiter un signe du vieillissement cutané ou des vergetures. Elle peut être également destinée à rendre moins visible des micro-vaisseaux sous-cutanés. Ladite composition peut également être destinée à prévenir, traiter ou atténuer une rougeur cutanée.

Les signes du vieillissement cutané englobent un amincissement de la peau, en particulier de l'épiderme, l'apparition d'un microrelief, l'apparition de ridules et/ou de rides sur la peau, y compris au niveau des lèvres et des paupières, un flétrissement ou un affaissement de la peau, une perte d'éclat de la peau, les cernes, un teint brouillé, une perte de densité de la peau, une perte de fermeté de la peau, une perte de tonicité de la peau, une perte d'élasticité de la peau, une altération de l'aspect lisse de la peau, et/ou une augmentation de la rugosité de la peau. Dans certains modes de réalisation, la composition est destinée à traiter ou prévenir une rougeur transitoire ou permanente, de préférence chez un type de peau choisi parmi le groupe constitué par une peau à tendance couperosique, une peau à tendance érythrosique et une peau à tendance érythro-couperosique.

La swertiamarine représente entre 0,001% à 5% en poids, de manière plus préférée entre 0,005% et 0,5% en poids du poids total de ladite composition.

Dans certains modes de réalisation, ladite composition comprend en outre, au moins un agent cosmétique additionnel, de préférence choisi dans le groupe constitué par les vitamines, les filtres et écrans solaires, les agents anti-âges, ou antirides, les antioxydants, les agents liftants, les agents raffermissants, les agents anti-tâches, les agents anti-rougeurs, les agents amincissants, les agents drainants, les agents hydratants, les agents apaisants, les agents gommants ou exfoliants, les agents matifiants, les agents séborégulateurs, les actifs éclaircissants, les actifs auto-bronzants, les accélérateurs de bronzage et leurs combinaisons.

Dans d'autres modes de réalisation, ladite composition peut se présenter sous différentes formes. Elle peut être choisie parmi le groupe constitué par les solutions aqueuses, les solutions hydroalcooliques, les émulsions huile-dans-eau (H/E) ou eau-dans huile (E/H) ou multiple (triple : E/H/E ou H/E/H), les nanoémulsions, en particulier des nanoémulsions H/E, dont la taille des gouttes est inférieure à 100nm, les gels aqueux, ou les dispersions d'une phase grasse dans une phase aqueuse à l'aide de sphérules, les suspensions, de préférence en milieux aqueux ou hydroalcoolique, les suspensions de liposomes, les poudres, les lotions, les laits, les crèmes, les onguents, les gels, les mousses, et les pommades.

Dans des modes de réalisation particulier, la composition se présente sous la forme d'un produit cosmétique, d'un produit de maquillage ou d'un produit d'hygiène corporelle, par exemple une lotion, un lait, un sérum, un gel aqueux ou huileux, une émulsion, une crème, un gel-crème, une eau de soin, une pommade, un baume, un fond de teint, un spray, un ombre à paupière, un stick, un rouge à lèvre, un gloss, un baume à lèvres, une mousse, un déodorant, un masque nourrissant, un gel douche, et un produit exfoliant ou gommant

La présente invention a également pour objet l'utilisation de la swertiamarine ou d'un extrait végétal enrichi en swertiamarine en tant qu'agent cicatrisant ou en tant qu'agent régénérateur de l'épiderme, dans le traitement ou la prévention d'une lésion de la peau ou d'une muqueuse telle que définie dans la revendication 11. La lésion de la peau ou de la muqueuse peut être notamment une coupure, des fissures, des plaies ou des micro-plaies, une gerçure, une crevasse, ou des fendillements.

Ladite swertiamarine ou ledit extrait sont destinés à être administrés par voie topique et, en option, en combinaison avec un autre principe actif, de préférence choisi parmi le groupe constitué par les agents apaisants, les agents hydratants, les agents anti-inflammatoires, les agents antioxydants, les agents cicatrisants, les agents désinfectants, les agents antimicrobiens (y compris les agents antibiotiques et les agents antifongiques) et leurs combinaisons.

Un objet supplémentaire selon l'invention est l'utilisation d'une composition pharmaceutique ou cosmétique dans le traitement d'une lésion de la peau ou d'une muqueuse, ladite composition comprenant de la swertiamarine ou un extrait végétal enrichi en swertiamarine en tant qu'agent cicatrisant ou agent régénérateur de l'épiderme. Ladite composition comprend 0,001% à 5% en poids, de manière plus préférée entre 0,005% et 0,5% en poids de swertiamarine. Ladite composition peut en outre comprendre un principe actif additionnel de préférence choisi dans le groupe constitué par les agents apaisants, les agents hydratants, les agents anti-inflammatoires, les agents antioxydants, les agents cicatrisants, les agents désinfectants, les agents antimicrobiens et leurs combinaisons

L'extrait végétal enrichi en swertiamarine peut être un extrait obtenu à partir d'une espèce de *Swertia,* de préférence *Swertia chirata* ou *Swertia milensis,* et qui comprend au moins 90% en poids de swertiamarine.

Enfin, l'invention a également pour objet une composition pour la préparation d'une composition pharmaceutique ou cosmétique, comprenant :
- de 0,1 à 20% de swertiamarine, ladite swertiamarine étant de préférence intégrée sous la forme d'un extrait végétal enrichi en swertiamarine, notamment un extrait obtenu à partir d'une espèce de *Swertia* telle que *Swertia chirata* ou *Swertia milensis,*
- de 50% à 99,9 % d'un véhicule, de préférence choisi parmi un agent de charge, un solvant aqueux, un solvant organique, de préférence un alcool inférieur tel que l'éthanol, le propanediol, le butylène glycol, la glycérine ou l'isopropanol, un agent lipophile et leurs mélanges, et
- en option, de 0,1 à 30% d'un excipient supplémentaire acceptable sur le plan pharmaceutique ou cosmétique, de préférence choisi parmi un agent de vectorisation, un agent antioxydant, un agent conservateur, un agent stabilisant, un agent épaississant, un émulsifiant, un agent gélifiant hydrophile ou lipophile, un parfum, une huile minérale ou organique, et leurs combinaisons,
les pourcentages étant exprimés en poids par rapport au poids total de la composition.

Cette composition dite « précurseur » est choisie parmi :
- une composition solide se présentant sous la forme d'une poudre dans laquelle la swertiamarine est absorbée sur un agent de charge, par exemple la maltodextrine, et
- une composition se présentant sous la forme d'une émulsion eau-dans-huile comprenant un agent lipophile, de préférence choisi parmi les acides gras, les esters d'acides gras tels que palmitate d'isopropyle et/ou les alcools gras et un agent émulsifiant ou de vectorisation choisi parmi les phospholipides, leurs dérivés hydrogénés et leurs mélanges.

### Présentation des figures

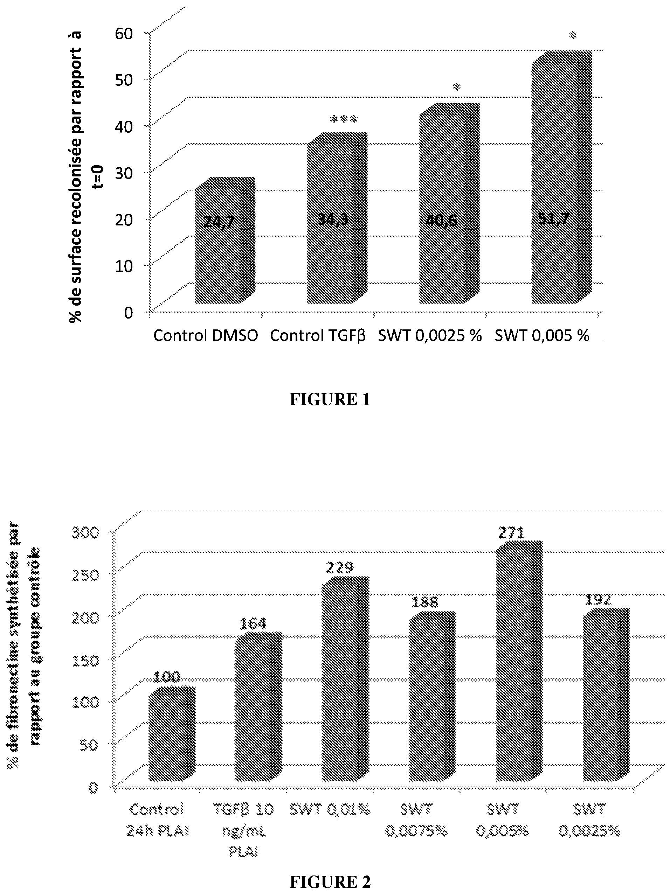

La **Figure 1** montre le pourcentage de recolonisation par les kératinocytes dans l'essai dit « scratch test » (voir Exemple 2) après incubation d'adipocytes avec de la swertiamarine (SWT) et conditionnement du milieu keratinocytaire avec le milieu adipocytaire, avec le TGF-β (produit de référence) ou en présence du solvant de dilution seulement (Control DMSO).
La **Figure 2** montre l'effet de la swertiamarine et du le TGF-β sur la synthèse de fibronectine par des fibroblastes normaux du derme par rapport à l'expérience contrôle (voir exemple 3).

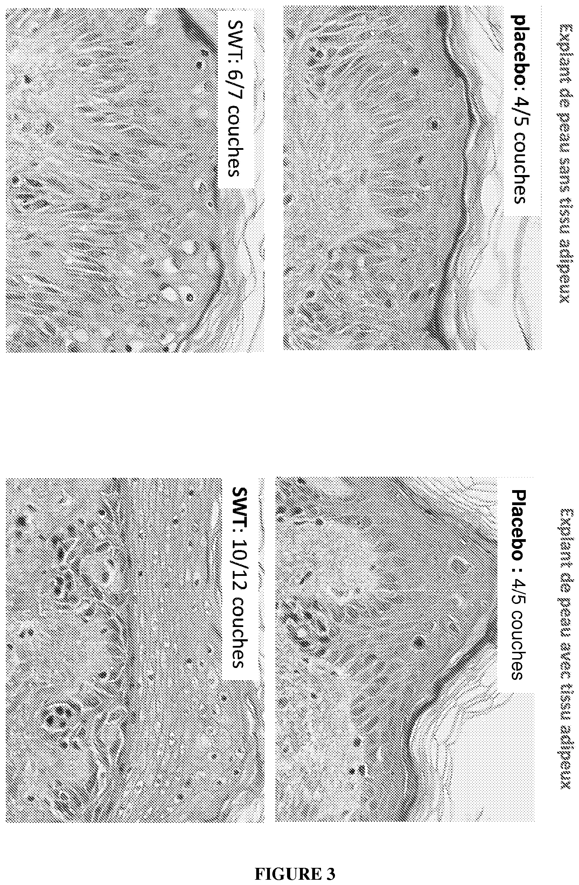

La **Figure 3** montre des coupes d'explant de peau avec ou sans tissu adipeux après incubation pendant 9 jours avec ou sans traitement avec de la swertiamarine.

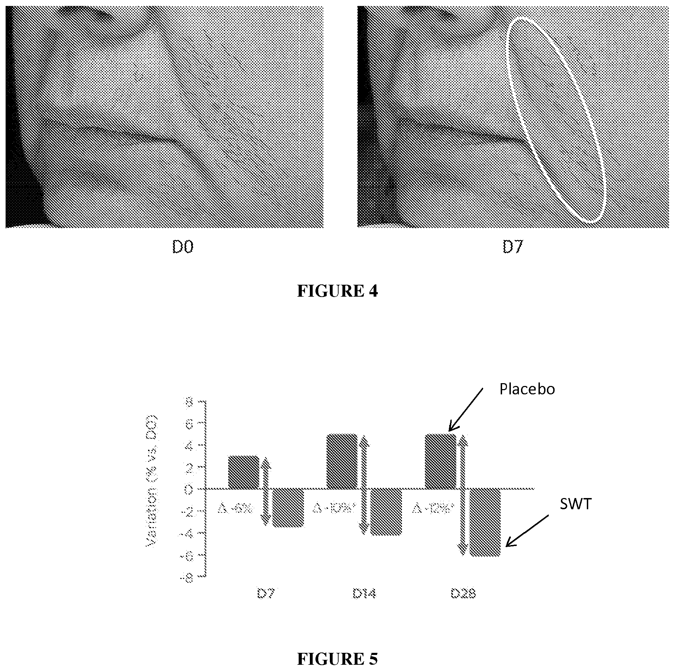

La **Figure 4** montre les photographies de la partie basse du visage d'une volontaire âgée de 51 ans à J = 0 (D0) et après 7 jours de traitement avec la crème SWT (D7) à 0,024% de swertiamarine, matin et soir. On note sur la photographie à J7 (D7), une diminution nette du volume des rides et une atténuation du sillon nasogénien (voir Exemple 6 - essai clinique n°1).
La **Figure 5** est un graphique montrant le pourcentage de variation du volume des rides par rapport à la valeur de référence mesurée à J=0, pour les peaux traitées par la crème placebo (Placebo) et les peaux traitées avec la crème « SWT » (SWT). On observe une augmentation du volume des rides pour les peaux traitées avec la crème placebo. En revanche, l'application biquotidienne de la crème « SWT» permet de diminuer le volume des rides au cours du temps, illustrant l'effet antiride de la swertiamarine (voir Exemple 6 - essai clinique n°2).

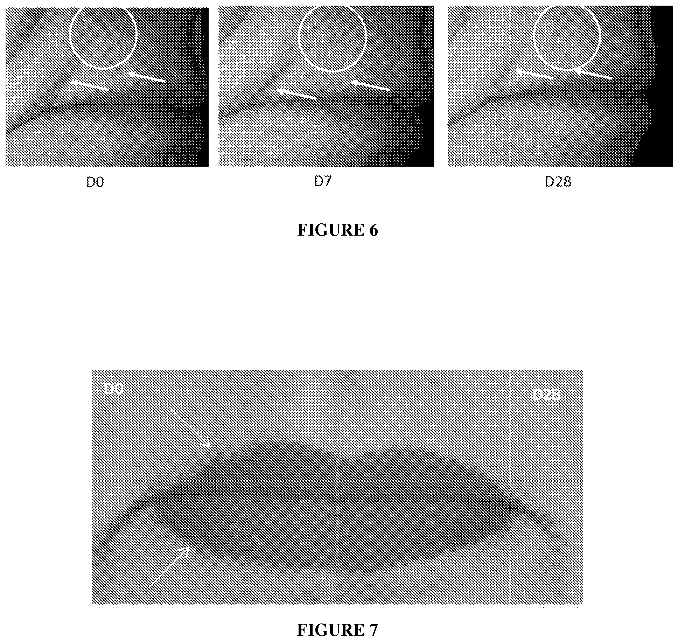

La **Figure 6** montre des macrophotographies du contour des lèvres d'une volontaire âgée de 64 ans à J = 0 (D0), J=7 (D7) et J=28 (D28) du traitement avec la crème SWT. On observe une nette diminution du relief des rides au niveau du contour supérieur de la lèvre sur les macrophotographies D7 et D28. La peau paraît plus lisse (voir Exemple 6 - essai clinique n°3).
La **Figure 7** montre la photographie du contour des lèvres d'une volontaire âgée de 50 ans à J = 0 (D0) et à J=28 (D28) du traitement avec la crème SWT. La migration du rouge à lèvres est significativement plus importante à J=0 (D0) qu'à J =28 (D28) (voir Exemple 6 - essai clinique n°4).

### Description de l'invention

Le genre *Swertia* appartient à la famille des Gentianacées et regroupent environ 150 espèces végétales, principalement en Asie et en Afrique. Les espèces de *Swertia* sont utilisées dans plusieurs médecines traditionnelles. A titre d'exemple, l'espèce *Swertia chirata* (aussi connue sous le nom de *Swertia chirayita*) est une espèce indigène de l'Himalaya utilisée dans l'Ayurveda, notamment pour ses propriétés hypoglycémique, antipyrétique, antiparasitaire, et antibactérienne ainsi que pour ses propriétés tonifiantes sur le système digestive.

En médecine traditionnelle, la plante entière est généralement utilisée pour préparer des poudres qui sont administrées sous forme d'infusion, de décoction ou de teintures. Des études ont montré que les plantes du genre *Swertia* comprennent un très grand nombre de composés de type xanthonoïde, alkaloïde, terpénoïde, flavonoïde, et iridoïde ayant potentiellement une activité biologique (Brahmachari et al., CHEMISTRY & BIODIVERSITY Vol. 1 (2004), 1627-1651). La swertiamarine (N°CAS : 17388-39-5) est un des nombreux composés qui a été isolé à partir des espèces de *Swertia,* notamment *Swertia Chirata* et *Swertia milensis.* Ce composé a été également isolé à partir d'autres plantes de la famille des Gentianacées par exemple certaines espèces du genre *Gentiana* et *Centaurium.* La formule chimique de la swertiamarine est comme suit :

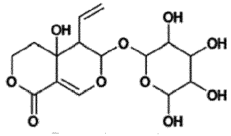

La swertiamarine est commercialisée sous forme purifiée (pureté d'au moins 95%) ou sous forme d'extraits enrichis. La swertiamarine a été décrite dans la littérature scientifique, entre autres, pour ses propriétés antidiabétique, anti-gastrique, analgésique, antimicrobienne et anti-cholestérol.

La présente invention concerne des nouvelles utilisations de la swertiamarine, ou des extraits végétaux enrichis en swertiamarine, dans le domaine cosmétique et thérapeutique.

De manière surprenante, le Demandeur a montré que la swertiamarine était capable de stimuler la prolifération des kératinocytes. En particulier, le Demandeur a mis en évidence que la swertiamarine induit la production du facteur de croissance de kératinocyte (KGF) par les cellules adipocytaires (Exemple 1). Le Demandeur a également montré sur la base d'un test de type « Scratch assay » qu'il était possible d'induire la prolifération des kératinocytes en les incubant en présence d'une culture d'adipocytes prétraités avec de la swertiamarine (Exemple 2). La swertiamarine a été également capable de promouvoir la croissance d'explants de peau humaine et d'augmenter l'épaisseur de l'épiderme (Exemple 3). Enfin, le Demandeur a mis en évidence que la swertiamarine était capable de stimuler de manière directe le métabolisme du derme, en particulier de stimuler la production de fibronectine, une glycoprotéine clé de la matrice extracellulaire (Exemple 4). De par sa capacité à induire la prolifération des kératinocytes et/ou la production de glycoprotéines constitutives du derme, la swertiamarine trouve une application directe en cosmétique, en particulier tant qu'agent antiride ou antiâge pour prévenir ou traiter les signes du vieillissement cutané. Le Demandeur a ainsi montré que l'application quotidienne d'une crème ayant une teneur d'environ 0,024% en poids de swertiamarine permettait d'atténuer de manière significative les rides et de diminuer la rugosité de la peau, en particulier dans la région des sillons nasogéniens et du contour des lèvres (voir Exemple 6). De manière plus générale, la swertiamarine peut être utilisée en tant qu'agent cosmétique pour traiter ou prévenir une altération non-pathologique de la peau nécessitant la restauration ou la régénération du tissu épidermique. A titre d'exemple, la swertiamarine peut être utilisée en tant qu'agent anti-rougeur.La swertiamarine trouve également des applications dans le domaine thérapeutique, notamment en tant qu'agent de la cicatrisation ou en tant qu'agent régénérateur de l'épiderme, par exemple dans le traitement ou la prévention des lésions de la peau ou des muqueuses.

### ▪ Utilisations de la swertiamarine selon l'invention

Dans le cadre de la présente, la swertiamarine peut être utilisée sous la forme d'un produit isolé ou sous la forme d'un extrait végétal enrichi en swertiamarine. L'extrait végétal enrichi en swertiamarine peut être obtenu à partir d'une plante appartenant à la famille des Gentianacées, telles que les espèces du genre *Swertia, Gentiana* ou *Centaurium.* De préférence, l'extrait végétal enrichi en swertiamarine est obtenu à partir d'une plante ou d'une partie d'une plante (feuille, fleur, tige et/ou graine) appartenant au genre *Swertia* telle que *Swertia Chirata* ou *Swertia milensis.* On entend par « *un extrait enrichi en swertiamarine* » un extrait comprenant au moins 90% en poids de swertiamarine. Il va de soi qu'un extrait enrichi de swertiamarine selon l'invention n'englobe pas une poudre obtenue directement par broyage de la plante entière de *Swertia* ou de l'une de ses parties.

Dans certains modes de réalisation, la swertiamarine est utilisée sous la forme d'un extrait végétal de *Swertia* comprenant au moins 80%, de préférence au moins 90% en poids de swertiamarine.

Dans certains modes de réalisation, il s'agit d'un extrait de *Swertia Chirata* ou de *Swertia milensis.* Un extrait végétal comprenant au moins 90% en poids de swertiamarine englobe un extrait végétal comprenant (ou ayant une teneur) d'au moins 91%, 92%, 93%, 94%, 95%, 96%, 97%, 98%, 99% voire 99,5% en poids de swertiamarine, ledit pourcentage se rapportant au poids total de l'extrait.

Dans certains modes de réalisation, l'extrait végétal enrichi en swertiamarine selon l'invention comprend au moins 80%, de préférence au moins 90% en poids de swertiamarine et au plus 20%, de préférence au plus 10% en poids de composés autres que la swertiamarine, en particulier des impuretés. Les impuretés ou les autres composés présents dans l'extrait selon l'invention peuvent provenir soit de la matrice végétale de départ, soit d'un ou plusieurs composés chimiques utilisés pendant le procédé d'extraction.

La préparation de tels extraits est décrite dans l'état de la technique. Plusieurs extraits adaptés à la mise en œuvre de la présente invention sont disponibles dans le commerce. Ces extraits peuvent être obtenus à partir toute partie d'une plante connue pour contenir de la swertiamarine, telle que les graines, les feuilles, les racines, les tiges ou encore les fleurs. Dans certains modes de réalisation préférés, l'extrait enrichi en swertiamarine est obtenu par un procédé d'extraction des feuilles d'une espèce de *Swertia,* notamment *Swertia Chirata.* Le procédé peut comprendre une étape de percolation ou une étape d'extraction par un solvant organique, notamment un alcool tel que l'éthanol. Il peut en outre comprendre une ou plusieurs étapes supplémentaires visant à augmenter la teneur en swertiamarine, par exemple une ou plusieurs étapes choisies parmi des étapes de concentration, de précipitation, de filtration, ou de chromatographie.

Selon un premier aspect, la présente invention a pour objet des utilisations, de préférence cosmétiques et non-thérapeutiques, de la swertiamarine. La swertiamarine peut être utilisée, de préférence à des fins cosmétiques, pour stimuler la formation ou la régénération de l'épiderme, en particulier stimuler la prolifération des kératinocytes. Dans certains modes de réalisation particulier, la swertiamarine, ou un extrait végétal enrichi en swertiamarine, tel qu'un extrait de *Swertia chirata,* peut être utilisé(e) à des fins cosmétiques, en tant qu'agent pour stimuler la prolifération des kératinocytes, en tant qu'agent pour augmenter ou restaurer l'épaisseur de l'épiderme, ou encore en tant qu'agent stimulant la formation ou la régénération de l'épiderme.

On entend par « *restaurer l'épaisseur de l'épiderme »* le fait d'augmenter l'épaisseur de l'épiderme, c'est-à-dire augmenter le nombre de couches de kératinocytes de l'épiderme de manière à obtenir une épaisseur d'épiderme équivalente à celui observée pour l'épiderme d'une peau jeune. La swertiamarine peut être également utilisée pour stimuler la production d'une protéine de la peau. On entend par « *protéine de la peau »* toute protéine constitutive de la matrice extracellulaire, en particulier le collagène et la fibronectine.

Selon un aspect supplémentaire, la swertiamarine peut être utilisée à des fins cosmétiques pour stimuler le métabolisme du derme, en particulier pour stimuler la production de fibronectine dans le derme.

La présente invention a également pour objet l'utilisation cosmétique de la swertiamarine pour promouvoir la régénération et/ou la restauration du derme et/ou de l'épiderme, ou encore pour lutter ou prévenir l'atrophie non-pathologique de l'épiderme.

En particulier, la swertiamarine peut être utilisée pour prévenir ou traiter une altération non-pathologique de la peau. On entend par « *altération non pathologique de la peau »* toute modification non-pathologique de l'aspect visuel ou des propriétés mécaniques de la peau. Les altérations non pathologiques de la peau peuvent résulter, notamment du vieillissement cutané, d'une fragilité ou d'une sensibilité de la peau (peau dite réactive) ou de l'exposition de la peau à certaines conditions extérieures.

Dans certains modes de réalisation, la swertiamarine peut donc être utilisée en tant qu'agent cosmétique antiâge ou antiride.

Au sens de l'invention, le terme *« peau »* désigne toute partie de la peau du corps humain, en particulier la peau du visage, y compris les lèvres et les paupières, le cou, la peau des mains et la peau des pieds.

Au sens de l'invention, on entend par « *signes du vieillissement cutané* » toute altération ou modification de l'aspect visuel ou des propriétés mécaniques de la peau, en particulier de l'épiderme, non-pathologique, résultant du vieillissement cutané, qu'il soit chronologique (chrono-vieillissement) et/ou du photo-induit (photo-vieillissement).

Ainsi, les signes du vieillissement englobent, sans y être limités, un amincissement de la peau, en particulier de l'épiderme, l'apparition d'un microrelief, l'apparition de ridules et/ou de rides sur la peau, y compris au niveau des lèvres et des paupières, un flétrissement ou un affaissement de la peau, une perte d'éclat de la peau, un teint brouillé, les cernes au niveau des yeux, une perte de densité de la peau, une perte de fermeté de la peau, une perte de tonicité de la peau, une perte d'élasticité de la peau, une altération de l'aspect lisse de la peau, et/ou une augmentation de la rugosité de la peau.

A titre d'exemples, de par ses propriétés sur la prolifération des kératinocytes et/ou sur le métabolisme du derme, la swertiamarine peut être utilisée dans le contexte cosmétique pour:
- stimuler le métabolisme de l'épiderme et/ou la régénération de l'épiderme, en particulier pour lutter contre le vieillissement de la peau, plus spécifiquement de l'épiderme;
- améliorer l'éclat du teint ou unifier le teint;
- limiter les taches pigmentaires,
- prévenir, atténuer ou traiter les cernes,
- rajeunir la peau vieillissante,
- prévenir, atténuer ou traiter les rides, et/ou les ridules, en particulier chez les peaux matures;
- lisser la peau, ou limiter sa rugosité ;
- corriger l'affinement de la peau lié à l'âge;
- prévenir ou traiter la sécheresse de la peau, de préférence en augmentant l'effet barrière de l'épiderme,
- maintenir ou améliorer les caractéristiques mécaniques de la peau, telles que la tonicité, la fermeté, la souplesse et/ou l'élasticité de la peau, et/ou
- Lisser et/ou restructurer les lèvres, et/ou pour les rendre plus douces, moins rugueuses avec un contour mieux défini.

Dans le cadre de la présente invention, on entend par « *prévenir un signe du vieillissement cutané* », le fait de prévenir, de ralentir ou de retarder l'apparition du signe du vieillissement cutané.

On entend par « *traiter un signe du vieillissement cutané* » le fait de corriger, d'atténuer, d'estomper, de rendre moins visible, de réduire l'apparence voire de faire disparaître le signe du vieillissement cutané.

Dans certains modes de réalisation, la swertiamarine est utilisée de manière cosmétique pour prévenir ou traiter un signe du vieillissement cutané, de préférence choisi parmi un amincissement de l'épiderme, l'apparition d'un microrelief, l'apparition de ridules et/ou de rides sur la peau, y compris au niveau des lèvres et des paupières, une perte d'éclat de la peau et une altération d'une propriété mécanique de la peau telle qu'une perte de densité, de fermeté, de souplesse et/ou d'élasticité.

A cet égard, la swertiamarine est particulièrement efficace pour prévenir ou traiter les rides et les ridules du visage, en particulier les rides verticales du visage, par exemple situées autour des lèvres ou au bas du visage dans la région des sillons nasogéniens.

On entend par « *lisser la peau* » le fait d'atténuer et/ou de corriger le relief de la peau, y compris des lèvres, ledit relief se présentant sous la forme de ride, de ridules et/ou vergetures, et peut être même consécutif à la présence de varices. De préférence, le relief de la peau se présente sous la forme de rides, de ridules et/ou d'une rugosité de la peau.

L'effet sur le signe du vieillissement cutané ou l'effet de lissage de la peau peut être évalué par l'une des méthodes décrites dans l'Exemple 6, notamment à l'aide d'un système d'imagerie faciale Visia-CR®, ou d'un système d'imagerie par projection de franges tel que le système Primos® 3D Pico en comparant une zone de peau traitée avec une composition comprenant la swertiamarine ou un extrait enrichi en swertiamarine *versus* une zone de peau traitée avec une crème placebo.

La swertiamarine peut être également utilisée, à titre cosmétique, pour prévenir ou traiter d'autres types altérations non-pathologiques de la peau impliquant un dysfonctionnement non-pathologique du renouvellement de l'épiderme. Il peut s'agir, à titre d'exemple de vergetures ou de rougeurs. Les vergetures peuvent être formées au cours de la ménopause, de la grossesse ou lors d'une perte ou d'une prise importante de poids. A titre d'exemple, la swertiamarine peut être utilisée en tant qu'agent anti-vergeture, par exemple pour réduire l'apparence des vergetures. La swertiamarine peut être également utilisée pour rendre moins visible les varices.

Avec le vieillissement cutané et l'exposition aux conditions extérieures, l'épiderme peut s'amincir et ne plus jouer parfaitement son rôle de barrière. La peau devient alors plus sensible et sujette aux rougeurs et à la sécheresse cutanée. Par ailleurs, l'amincissement de la peau peut rendre les micro-vaisseaux sous-cutanés plus visibles, ce qui peut accentuer l'intensité des rougeurs.

Ainsi, de par sa capacité à induire la prolifération des kératinocytes, la swertiamarine peut également être utilisée, de préférence à titre cosmétique et non-thérapeutique, en tant qu'agent anti-rougeur. Au sens de l'invention, un « *agent anti-rougeur »* englobe :
- un agent capable de prévenir, traiter ou d'atténuer les rougeurs,
- un agent capable de diminuer la tendance à rougir d'une peau telle qu'une peau réactive, fragile ou sensible, en particulier en réponse à un facteur extérieur, ou encore
- un agent capable d'atténuer l'aspect rouge associé à des micro-vaisseaux de surface ou à une anomalie du système vasculaire sous cutané ou rendre moins visible ladite anomalie.

Au sens de l'invention, « *une anomalie du système vasculaire sous cutané* » englobe, entre autres, une télangiectasie et un angiome.

Au sens de l'invention, le terme *«rougeur »* englobe les rougeurs et les échauffements cutanés, en particulier au niveau du visage par exemple au niveau des joues, du nez et du menton. Il peut s'agir de rougeurs ou d'échauffements (également appelés « flush ») épisodiques, transitoires ou passagers qui peuvent être induits ou favorisés par un facteur extérieur, en particulier par une condition climatique telle que l'exposition aux UV, le vent, une température ambiante trop basse ou trop élevée ou un changement brusque de températures, par l'ingestion d'un aliment, par exemple d'une boisson chaude, un alcool ou des épices, ou encore par un stress ou une émotion.

Il peut s'agir également de rougeurs installées ou permanentes, par exemple associées à la présence de micro-vaisseaux de surface, à un angiome ou à une télangiectosie. Enfin il peut s'agir de rougeurs passagères ou permanentes associées à une couperose ou à une érythrose. De préférence, le terme « *rougeur »* se réfère aux rougeurs associées à la présence de micro-vaisseaux de surface, à une couperose ou à une érythrose.

Dans certains modes de réalisation, la rougeur est due à des micro-vaisseaux de surface associés à une couperose ou à une érythrose. Dans d'autres modes de réalisation, la rougeur est due à des micro-vaisseaux de surface qui n'est pas associés à une couperose ou à une érythrose.

Dans d'autres modes de réalisation, la swertiamarine est utilisée pour traiter ou prévenir l'apparition de rougeurs transitoires ou permanentes chez un type de peau choisi parmi une peau sensible, une peau fragile, une peau réactive, une peau intolérante, une peau à tendance couperosique, une peau à tendance érythrosique ou une peau à tendance érythro-couperosique

On entend par une peau à tendance érythrosique ou couperosique une peau susceptible d'être affectée par une couperose ou une érythrose.

De par son action générale sur les rougeurs, les tâches et les cernes, la swertiamarine peut être également utilisée en tant qu'agent unificateur de teint.

La swertiamarine peut être également utilisée pour rendre moins visible, à la surface de la peau, une varice, des micro-vaisseaux de surface, un angiome ou une télangiectosie. Les micro-vaisseaux de surface peuvent être congénitaux, ou apparaitre consécutivement au vieillissement cutané ou au développement d'une couperose. Cette action de la swertiamarine repose notamment sur sa capacité à stimuler le derme et l'épiderme, plus particulièrement sur sa capacité à promouvoir la prolifération des kératinocytes et donc restaurer ou augmenter l'épaisseur de l'épiderme.

Dans les utilisations cosmétiques selon l'invention, la swertiamarine est présente, à titre d'agent actif, dans une composition, de préférence cosmétique.

La swertiamarine peut être incorporée dans la composition sous une forme purifiée ou sous la forme d'un extrait végétal enrichi en swertiamarine tel que défini précédemment. Dans certains modes de réalisation, la swertiamarine est présente sous la forme d'un extrait végétal comprenant au moins 90% en poids de swertiamarine, ledit extrait végétal étant de préférence un extrait de *Swertia chirata* ou de *Swertia milensis.*

Cette composition est destinée à être administrée par voie topique. Typiquement, la composition est destinée à être appliquée sur la peau, par exemple sur la peau des mains ou du visage, y compris les lèvres ou les paupières.

La swertiamarine (ou l'extrait végétal enrichi en swertiamarine) est présente dans la composition en une quantité comprise allant de 0,001% à 5% en poids, de manière plus préférée entre 0,001% et 0,5% voire de 0,005% à 0,1%, les pourcentages étant exprimés par rapport au poids total de la composition cosmétique. A titre d'exemple, la swertiamarine peut être présente à hauteur de 0,01% à 0,1% en poids, par exemple à hauteur de 0,015% à 0,040% en poids de la composition cosmétique. La composition peut, en outre comprendre, un ou plusieurs principes actifs additionnels. De préférence, le ou les principes actifs additionnels exercent un effet cosmétique.

On entend par « *principe actif à effet cosmétique, agent actif à effet cosmétique, ou actif à effet cosmétique* » un composé capable d'exercer au moins un effet cosmétique sur la peau ou ses annexes. On entend par « *effet cosmétique* » tout effet non-thérapeutique visant à modifier et/ou améliorer l'aspect de la peau ou des muqueuses comme les lèvres, à les protéger des agressions externes (soleil, vent, humidité, sécheresse, produits chimiques), ou encore à prévenir et/ou à corriger les phénomènes liés à leur vieillissement.

Ainsi, dans certains modes de réalisation, la swertiamarine peut être présente dans une composition qui comprend, en outre, un principe actif à effet cosmétique additionnel. Ce principe actif à effet cosmétique peut être choisi parmi le groupe constitué par les vitamines, les filtres et écrans solaires, les agents anti-âges, ou antirides, les agents anti-rougeurs, les antioxydants, les agents liftants, les agents raffermissants, les agents hydratants, les agents apaisants, les agents gommants ou exfoliants, les agents matifiants, les agents séborégulateurs, les actifs éclaircissants, les actifs anti-tâches, les agents amincissants, les agents drainants, les actifs auto-bronzants, les accélérateurs de bronzage et leurs combinaisons. Notamment, la composition cosmétique peut comprendre des tocophérols, et/ou des extraits de plantes comme des extraits de graines de lin, des extraits d'exopolysaccharides de *Vibrio,* des peptides comme le trifluoroacetyl tripeptide-2.

De préférence, la composition cosmétique peut comprendre un actif choisi parmi un agent antiride, un agent anti-âge, un agent anti-rougeur, un agent antioxydant, un actif hydratant, un agent apaisant, un agent séborégulateur, un agent anti-tâches et leurs combinaisons. De manière encore plus préférée, l'agent actif à effet cosmétique additionnel est choisi parmi un agent antiride, un agent anti-âge, un agent antioxydant, un agent hydratant, un agent liftant, un agent raffermissant et leurs combinaisons.

Le ou les principes actifs additionnels à effet cosmétique sont typiquement présents à hauteur de 0,0001% à 10% en poids de la composition.

A titre d'exemple d'agents hydratants, on peut citer l'urée, l'acide pidolique (PCA) et ses dérivés en particulier ses sels tels que l'arginine PCA, le chitosan PCA, ses sels de cuivre (Cuivre PCA), de magnésium (magnésium PCA), de sodium (sodium PCA) ou de zinc, l'éthylhéxyl PCA, le gluconate de calcium, l'acide hyaluronique et ses sels et autres glycosaminoglycanes, le frucose, le glucose, l'isomaltose, le lactose, le tréhalose, le polydextrose, le saccharose (Sucrose), le maltitol, le mannitol, le sorbitol, le xylitol et les autres hydrates de carbones et dérivés, les polyéthylène glycols tels que les PEG-7, PEG-8, PEG-10, PEG-12 ou PEG-14, la glycérine , le propylène glycol, le butylène glycol, la betaïne, la citrulline, le collagène et ses dérivés, l'histidine, les hydrolysats de soie, de kératine ou de soja, les extraits de plante riches en polysaccharides et/ou polyphénols, par exemple les extraits d'Aloès, bleuet (*Centaurea cyanus*), et les combinaisons de ceux-ci.

A titre d'exemple d'agents anti-âge, antirides ou liftants, on peut citer l'acide ascorbique et ses dérivés comme l'ascorbyl phosphate de magnésium, les glycosaminoglycanes et leurs dérivés, le ribose, le sorbitol, les polysaccharides de *Cyathea,* le collagène, les extraits de graines de lin (*Linum usitatissimum*), les peptides comme le Caprooyl-Tetrapeptide-3 et le trifluoroacétyl tripeptide-2, les extraits de *Polygonum aviculare,* les extraits d'algues brunes, en particulier d'*Ascophyllum nodosum,* les extraits de fougères, en particulier de *Cyathea Cumingii*

A titre d'exemple d'agents apaisants, on peut citer l'allantoïne, les extraits d'aloès, de bouleau (par exemple *Betula alba*), d'épilobe (*Epilobium angustifolium*), de châtaignier (par exemple *Castenea sativa*), de bleuet (par exemple *Centaurea cyanus*), de centella (par exemple *Centella asiatica*), de prêle (par exemple *Equisetum arvense*), de fenouil (par exemple *Foeniculum vulgare*), d'hamamélis (par exemple *Hamamelis virginiana*), de lierre (par exemple *Hedera helix*), d'*habiscus sabdariffa,* de lis (par exemple *Lilium candidum*), de mauve (par exemple *Malva sylvestris*), de mélisse (par exemple *Melissa officinalis*), de scutellaire (par exemple *Scutellaria baicalensis*), de mimosa (par exemple *Mimosa tenuiflora*), de potentille (par exemple *Potentilla erecta*), un extrait d'oligosaccharides ou un oligosaccharide, par exemple de lin, les peptides comme le palmitoyl tripeptide-8, les polysaccharides obtenus par biotechnologie comme l'extrait de ferment d'*Alteromonas* et les combinaisons de ceux-ci.

A titre d'exemple d'agents antioxydants, on peut citer le HMR (hydroxy méthyl résorcinol), l'acide ascorbique et ses dérivés, la vitamine B9, le chlorhydrate d'histidine, ou un extrait d'épilobe (*Epilobium augustifolium*). Les principes actifs à effet antioxydant et de type vitamine sont généralement utilisés en un pourcentage massique d'au moins 1% par rapport au poids total de la composition cosmétique.

A titre d'exemple d'agents séborégulateurs, on peut citer les lignanes de lin, la poudre de riz, le gluconate de zinc, la sarcosine, un extrait de *Cinnamomum zeylanicum bark,* un extrait d'avocat et les combinaisons de ceux-ci.

A titre d'agents anti-rougeurs, on peut citer les saponines, les flavonoïdes, les ruscogénines, les esculosides, et les extraits les contenant, par exemples les extraits de *Ruscus,* ainsi que certaines huiles essentielles par exemple de lavande ou de romarin.

A titre d'exemple d'agents anti-tâches, on peut citer des extraits comme la réglisse (*Glycyrrhyza glabra*), l'extrait de jackfruit (*Artocarpus heterophyllus*), les extrait de Rumex (*R.occidentalis*), les extraits de plante appartenant au genre citrus, les extraits de plantes riches en stibènes comme le resveratrol, les petides comme l'oligopeptide-68, le nonapetide-1, l'arbutine, l'acide kojique, le magnésium ascorbyl phosphate et les combinaisons de ceux-ci.

L'invention a également pour objet un procédé cosmétique pour traiter, atténuer ou prévenir un signe du vieillissement cutané, une rougeur ou un échauffement cutané chez un individu, ledit procédé comprenant l'administration d'une quantité cosmétiquement efficace de swertiamarine, de préférence par voie topique.

Un autre objet selon l'invention est un procédé cosmétique pour lisser la peau chez un individu, par exemple pour corriger ou atténuer les rides, les ridules ou les vergetures, ledit procédé comprenant l'administration d'une quantité cosmétiquement efficace de swertiamarine, de préférence par voie topique.

Un autre objet selon l'invention est un procédé cosmétique pour rendre moins visible, ou améliorer l'aspect, une varice, des micro-vaisseaux de surface, un angiome ou une télangiectosie chez un individu, ledit procédé comprenant l'administration d'une quantité cosmétiquement efficace de swertiamarine, de préférence par voie topique, sur la zone cutanée où la varice, les micro-vaisseaux de surface, l'angiome ou la télangiectosie est/sont visible(s).

Comme précisément indiqué, la swertiamarine est typiquement administrée sous la forme d'une composition, ladite composition étant, de préférence, cosmétique et appliquée sur la peau, par exemple au niveau du visage, du cou ou des mains.

Selon un aspect supplémentaire, la présente invention a pour objet l'utilisation de la swertiamarine dans le domaine de la cicatrisation des lésions de la peau ou des muqueuses. Ainsi, la swertiamarine peut être utilisée pour favoriser la cicatrisation, en particulier pour favoriser la régénération de l'épiderme, la reconstruction de l'épiderme et/ou promouvoir la ré-épithélisation de la peau ou des muqueuses atteintes par une lésion. Il s'agit de préférence d'une utilisation thérapeutique de la swertiamarine. Plus généralement, la swertiamarine peut être utilisée pour prévenir ou traiter une lésion de la peau ou des muqueuses. La swertiamarine peut être ainsi utilisée à des fins thérapeutiques en tant qu'agent cicatrisant ou agent de ré-épithélisation ou de régénération de l'épiderme.

Comme mentionné ci-avant, la swertiamarine peut être utilisée sous la forme d'un produit isolé ou sous la forme d'un extrait végétal enrichi en swertiamarine, de préférence obtenu à partir d'une plante appartenant au genre *Swertia* telle que *Swertia Chirata* ou *Swertia milensis.*

La lésion peut correspondre à un traumatisme de la peau ou de la muqueuse, par exemple, une coupure, une griffure, une brûlure, y compris une brûlure chimique, thermique ou de contact, une irritation, un érythème, ou encore une cloque ou une ampoule. Selon l'invention, la lésion résulte d'une exposition de la peau ou d'une muqueuse à des agressions climatiques telles que le vent ou les fortes variations de températures, à certains produits chimiques tels que les détergents, à certains agents thérapeutiques tels que les agents anticancéreux ou anti-acnéiques, à une radiothérapie, au laser ou encore à certains procédés cosmétiques tels que le peeling ou la dermo-abrasion.

La lésion de la peau ou de la muqueuse peut correspondre à des fissures superficielles ou à des fissures plus profondes telles que des gerçures ou des crevasses.

Dans certain mode de réalisation, la swertiamarine peut être utilisée pour traiter une lésion de la peau ou d'une muqueuse telle qu'une coupure, une brûlure, une irritation, une ampoule, une cloque, des fissures, des micro-plaies, une gerçure, une crevasse, ou des fendillements. De préférence, la lésion de la peau ou d'une muqueuse est choisie parmi une coupure, des fissures, une plaie, des micro-plaies, une gerçure, une crevasse, ou des fendillements. A titre d'exemple, il peut s'agir de fissures ou de gerçures au niveau des lèvres ou des mains ou encore de fendillements ou de crevasses au niveau des talons.

Pour la mise en œuvre des utilisations thérapeutiques selon l'invention, la swertiamarine (ou l'extrait végétal enrichi en swertiamarine) peut être incorporée dans n'importe quel type de composition, qu'elle soit cosmétique ou pharmaceutique. Comme dans le cas des utilisations cosmétiques, la composition est de préférence destinée à être administrée par voie topique, par exemple à être appliquée sur la peau ou sur la muqueuse à traiter. La swertiamarine est présente dans la composition en une quantité comprise allant de 0,001% à 5% en poids, de manière plus préférée entre 0,001% et 0,5% voire de 0,005%% 0,1%, les pourcentages étant exprimés par rapport au poids total de la composition cosmétique ou thérapeutique. A titre d'exemple, la swertiamarine peut être présente à hauteur de 0,01% à 0,1% en poids, par exemple à hauteur de 0,015% à 0,040% en poids de la composition cosmétique ou thérapeutique.

La composition peut en outre comprendre un ou plusieurs principes actifs additionnels choisis parmi les actifs à effet cosmétique et/ou parmi les actifs à effet thérapeutique. A titre d'exemple, le principe actif additionnel peut être choisi dans le groupe constitué par les agents apaisants, les agents hydratants, les agents anti-inflammatoires, les agents antioxydants, les agents cicatrisants, les agents favorisant la régénération de l'épiderme, les agents désinfectants, les agents antimicrobiens par exemple les antifongiques, les agents désinfectants ou les agents antibiotiques et leurs combinaisons.

Typiquement, les agents apaisants, les agents hydratants, et les agents antioxydants précédemment listés pour les utilisations cosmétiques selon l'invention peuvent être également utilisés dans le cadre des utilisations dans le domaine de la cicatrisation selon l'invention.

A titre d'exemple d'agents favorisant la régénération de l'épiderme, on peut citer le madécassol, l'oxaceprol, un extrait de *Calendula officinalis,* un extrait de millepertuis (*Hypericum perforatum*), un extrait d'achilée (*Achilea millefolium*), un extrait de *Ledum palustre,* l'oxyde de zinc, le baume du perou, la vitamine A (retinol), le dexpanthenol.

A titre d'exemple d'agents anti-inflammatoires, on peut citer les corticoïdes, l'acide salicylique, et les anti-inflammatoire non-stéroïdiens

A titre d'agents désinfectants, on peut citer la chlorhexidine, les ammoniums quaternaires ; le triclocarban, les dérivés anioniques, les organo-mercuriels, les sels de cuivre et ou de zinc, les dérivés de l'acide para-hydroxybenzoique, l'hexamidine et ses dérivés, les dérivés iodés.

A titre d'agents antibiotiques, on peut citer les macrolides, l'acide fusidique, les aminosides, la rifamycine, les sulfamides.

A titre d'agents antifongiques, on peut citer l'imidazole et ses dérivés, la terbinofine, le sulfure de sélénium

Le ou les principes actifs additionnels sont typiquement présents à hauteur de 0,0001% à 10% en poids de la composition.

Selon un autre aspect, la présente invention a pour objet l'utilisation de la swertiamarine en combinaison avec un agent actif choisi de préférence parmi les agents apaisants, les agents hydratants, les agents anti-inflammatoires, les agents antioxydants, les agents cicatrisants, les agents antimicrobiens y compris les agents antifongiques, les agents désinfectants ou les agents antibiotiques, dans le traitement ou la prévention d'une lésion de la peau ou d'une muqueuse ou pour promouvoir la cicatrisation. La swertiamarine et l'agent actif additionnel peuvent être administrés de manière simultanée, successive ou séparée dans le temps.

Selon un aspect supplémentaire, la présente invention a pour objet une méthode pour traiter ou prévenir une lésion de la peau ou d'une muqueuse chez un patient, ladite méthode comprenant l'administration audit patient d'une quantité efficace de swertiamarine, de préférence par voie topique.

Selon un autre aspect, un objet supplémentaire selon l'invention est l'utilisation de la swertiamarine pour la préparation d'une composition, de préférence cosmétique ou pharmaceutique, destinée au traitement ou à la prévention d'une lésion de la peau ou d'une muqueuse.

Dans les méthodes et utilisations selon l'invention, la dose à administrer et la fréquence d'administration de la swertiamarine varient en fonction de l'effet cosmétique ou de l'effet thérapeutique recherché, des caractéristiques de l'individu en particulier de son sexe, de son âge et de son type de peau. Pour les utilisations cosmétiques selon l'invention, la posologie peut varier selon le ou les signes de vieillissement que l'on souhaite prévenir ou traiter. Pour les utilisations thérapeutiques selon l'invention, la posologie peut varier selon le type et la gravité de la lésion que l'on souhaite traiter.

Typiquement, dans le cadre d'une utilisation cosmétique selon l'invention, la swertiamarine peut être appliquée, sur la zone à traiter, une à deux fois par jour, typiquement le matin et/ou le soir, pendant plusieurs semaines consécutives voire plusieurs mois, par exemple au moins pendant 1 mois, par exemple au moins pendant 3 mois. Dans le cadre d'une utilisation thérapeutique selon l'invention, la swertiamarine peut être appliquée, sur la plaie à traiter à raison de deux à trois fois par jour, de préférence jusqu'à cicatrisation complète, par exemple durant une semaine.

A titre d'exemple particulier de méthodes cosmétiques selon l'invention, on peut citer une méthode pour prévenir, atténuer ou traiter un signe du vieillissement cutané chez un individu, ladite méthode comprenant l'application d'une composition cosmétique comprenant de la swertiamarine ou un extrait végétal enrichi en swertiamarine sur la peau au moins une fois par jour, pendant au moins sept jours.

De préférence, le signe du vieillissement est choisi parmi un amincissement de l'épiderme, une ride ou ridule, l'apparition d'un microrelief, notamment d'une rugosité, et les combinaisons de ceux-ci.

La zone de peau à traiter est de préférence choisie parmi les mains, le cou, le visage ou une partie du visage tel que le contour des yeux, le contour des lèvres et la région des sillons nasogéniens.

Par exemple, la composition cosmétique peut être appliquée uniformément sur tout le visage ou sur une zone particulière du visage.

Une composition cosmétique selon l'invention peut être appliquée typiquement 1 à 2 fois par jour, de préférence matin et soir.

L'application de la cosmétique est réalisée au moins pendant 7 jours, de préférence pendant au moins 28 jours, voire pendant plusieurs mois.

Comme décrit ci-après, la composition cosmétique peut être sous toute forme adaptée à une application sur la peau, notamment sous la forme d'une crème, d'un baume, ou d'un gel.

La composition cosmétique peut comprendre de 0,001% à 0,1% en poids, par exemple de 0,015% à 0,040% en poids de swertiamarine.

La méthode cosmétique selon l'invention peut en outre comprendre une phase d'entretien qui suit une première phase dite d'attaque. La phase d'entretien se caractérise par l'utilisation d'une composition cosmétique comprenant une teneur plus faible en swertiamarine ou par une fréquence d'administration de la composition cosmétique plus faible.

Par exemple, pour une méthode dans laquelle, la composition cosmétique selon l'invention peut être appliquée à raison de deux fois par jour dans la phase d'attaque, le nombre d'applications peut être réduit à une fois par jour dans la phase d'entretien.

Le cas échéant, la phase d'attaque et la phase d'entretien se caractérisent par la même fréquence journalière d'application, mais la composition cosmétique de la phase d'entretien comprend une teneur en poids de swertiamarine inférieure d'au moins un facteur 1,5, par exemple au moins un facteur 2, 3, 4, 5, 6, 8, ou 10, à la teneur en swertiamarine de la composition cosmétique utilisée dans la phase d'attaque.

Les compositions adaptées pour la mise en œuvre des utilisations et des procédés cosmétiques ou thérapeutiques selon l'invention sont détaillées ci-après.

### ▪ Compositions contenant la swertiamarine selon l'invention

Les compositions selon l'invention comprennent de 0,001 % à 5% en poids de swertiamarine et des excipients acceptables sur le plan pharmaceutique ou cosmétique.

Comme mentionné ci-avant, la swertiamarine peut être introduite dans ladite composition sous forme purifiée ou isolée ou sous la forme d'un extrait végétal enrichi en swertiamarine.

Le ou les excipients acceptables sur le plan pharmaceutique ou cosmétique peuvent être choisis parmi les agents diluants, les agents dispersants, les agents gélifiants, les émollients, les agents de vectorisation comme les polymère polycationiques ou les phospholipides, les gommes, les résines, les solvants en particulier les alcools inférieurs notamment l'éthanol, l'isopropanol, le dipropylène glycol, le butylène glycol, le propanediol, le glycérol, le sorbitol, et le propylène glycol, les charges telles que les amidons modifiés et polymérisés, le dioxyde de titane, ou un stéarate métallique, les conservateurs, les huiles essentielles, les agents nacrés, les colorants, les absorbeurs d'odeur, les agents régulateurs du pH ou les agents neutralisants, les agents lubrifiants, les agents épaississants, les tensio-actifs dont les tensio-actifs anioniques, cationiques, amphotères ou non ioniques, les humectants, les agents mouillants, les agents dispersants, les agents aromatisants ou parfumant, les pigments organiques ou encore minéraux comme les oxydes de fer, les agents huileux comme les huiles ou des graisses d'origine végétale, les graisses d'origine animale, les huiles de synthèse comme la vaseline, les huiles de silicone (cyclométhicone), les esters d'alcool gras (alcool cetylique), des huiles fluorées, des des cires, des argiles modifiées, des bentones, des sels métalliques d'acide gras, de la silice hydrophobisée, des polyethylènes, du mica, les agents conservateurs, les agents antimicrobiens, des véhicules tels qu'une eau minérale, thermale ou florale, et/ou d'autres substances utilisées couramment en formulation dans le domaine cosmétique ou pharmaceutique.

Dans certains modes de réalisation, la swertiamarine est formulée à l'aide d'un système de vectorisation. On entend par « *système de vectorisation* » un système supramoléculaire dont le but est de promouvoir la pénétration de la swertiamarine à travers la peau, de préférence à travers l'épiderme voire le derme. Dans un mode de réalisation préféré, la swertiamarine est encapsulé dans le système de vectorisation.

Le système de vectorisation peut être choisi parmi le groupe constitué par les micelles, les liposomes, y compris les liposomes unilamellaires ou multilamellaires, les niosomes, les éthosomes, les systèmes lamellaires, les nanosomes, les vésicules lipidiques ou polymériques, les nanosphères, les micro ou nano-particules de polymères naturels ou non, les hydrogels. De préférence, la swertiamarine est encapsulée dans un système de vectorisation choisi parmi les liposomes et les systèmes lamellaires. Des exemples particuliers de liposomes et de systèmes lamellaires pour la mise en œuvre de la présente invention sont décrits, entre autres, dans les demandes de brevet français n° 1358589 et n°1262303 déposées au nom du Demandeur.

A titre d'exemple, le système de vectorisation peut être un système lamellaire de bicouches lipidiques en phase aqueuse, ledit système lamellaire comprenant un mélange de phospholipide, d'acide gras et d'alcool gras ayant de préférence un rapport massique « phospholipide/acide gras » de 0,5 à 1,5 et un rapport massique « alcool gras/acide gras » de 2 à 4.

L'alcool gras peut être choisi dans le groupe constitué par l'alcool caprylique (octan-1-ol), l'alcool caprique (décan-1-ol), l'alcool laurylique (dodécan-1-ol), l'alcool myristylique (tétradécan-1-ol), l'alcool palmitique (hexadécan-1-ol), l'alcool stéarylique (octadécan-1-ol), l'alcool béhénique (docosan-1-ol) et leurs mélanges.

L'acide gras peut être choisi dans le groupe constitué par l'acide caprylique, l'acide caprique, l'acide laurique, l'acide myristique, l'acide palmitique, l'acide stéarique, l'acide béhénique et leurs mélanges. Le phospholipide peut être choisi parmi le groupe constitué par la phosphatidylcholine, la phosphatidylsérine, la phosphatidyléthanolamine, le phosphatidylglycérol, l'acide phosphatidique, le phosphatidylinositol, les lysophospholipides, leurs dérivés hydrogénés, et leurs mélanges.

A titre d'exemple supplémentaire, le système de vectorisation peut être un liposome comprenant au moins un phospholipide et au moins un glycolipide de préférence selon un rapport massique « phospholipide/glycolipide » allant de 0,5 à 30, de préférence de 1 à 10. De préférence, le motif glucidique du glycolipide comprend un oligomère ou un polymère de fructose (fructane) tels qu'un radical inuline ou levane. Le glycolipide est choisi parmi la stéaroyl inuline, l'inuline lauryle carbamate, la palmitoyl inuline, l'undécylénoyl inuline et leurs mélanges. Le phospholipide peut être tel que défini précédemment pour le système lamellaire.

La composition selon l'invention peut être sous toute forme connue. De préférence, il s'agit d'une composition ayant une forme adaptée à l'administration topique. Elle peut se présenter sous la forme de solutions aqueuses, hydroalcooliques, d'émulsions huile-dans-eau (H/E) ou eau-dans huile (E/H) ou multiple (triple : E/H/E ou H/E/H), des nanoémulsions, en particulier des nanoémulsions H/E, dont la taille des gouttes est inférieure à 100nm, de gels aqueux, ou de dispersions d'une phase grasse dans une phase aqueuse à l'aide de sphérules, de suspensions, de préférence en milieux aqueux ou hydroalcoolique ou encore d'une poudre.

La composition selon l'invention peut se présenter sous la forme d'une lotion, d'un lait, d'une crème, d'un onguent, d'un gel, d'une mousse, d'une solution, d'une pommade. La composition peut être également incluse dans un système plus complexe, par exemple au sein d'un pansement, ou d'un patch, d'un tissu imbibé, ou se présenter sous la forme d'un comprimé ou film mucoadhésif.

De préférence, la composition selon l'invention est une composition pharmaceutique ou cosmétique, de manière préférée une composition cosmétique et de manière encore plus préférée une composition dermocosmétique.

Ainsi, la composition selon l'invention peut se présenter également sous la forme d'un produit cosmétique de tout type. Il peut s'agir d'un soin cosmétique ou d'un produit de maquillage ou d'hygiène corporelle, par exemple une lotion, un lait, un sérum, un gel aqueux ou huileux, une émulsion, une crème, un gel-crème, une eau de soin, une pommade, un baume, un fond de teint, un spray, un ombre à paupière, un stick, un rouge à lèvre, un gloss, une mousse, un déodorant, un masque nourrissant, un gel douche, et un produit exfoliant ou gommant. A titre illustratif et non-limitatif, la swertiamarine peut être présente à titre d'agent cicatrisant ou régénérateur de l'épiderme dans une crème réparatrice pour les mains abimées ou encore dans un baume pour lèvres gercées. La swertiamarine peut également être présente à titre d'agent antiride ou antiâge dans une crème de jour destinée aux peaux matures ou âgées. A titre d'exemple supplémentaire, la swertiamarine peut être présente à titre d'agent pour lisser et/ou restructurer les lèvres dans un rouge à lèvres, un gloss ou un baume à lèvres. La swertiamarine peut être également présente en tant qu'agent antiride ou agent lissant dans un soin de contour des yeux ou des lèvres. La swertiamarine peut être également présente en tant qu'agent anti-rougeur dans une crème de jour destinée à rendre moins visible les micro-vaisseaux de surface..

Alternativement, la composition selon l'invention peut se présenter sous la forme d'un médicament destiné à être appliqué sur la peau ou une muqueuse, par exemple un gel ou une crème.

Les compositions cosmétiques ou pharmaceutiques selon l'invention peuvent être préparées selon des méthodes classiques, bien connues de l'homme du métier.

### ▪ Composition « précurseur » selon l'invention

Selon un aspect supplémentaire, l'invention a pour objet une composition destinée à être incorporée dans une composition cosmétique ou pharmaceutique. Cette composition correspond donc à une composition « *précurseur ».*

De préférence, cette composition pour la préparation d'une composition cosmétique ou pharmaceutique comprend :
- de 0,1 à 20%, de préférence de 0,5 à 10%, de swertiamarine, ladite swertiamarine étant de préférence intégrée sous la forme d'un extrait végétal enrichi selon l'invention,
- de 50% à 99,9 % d'un véhicule, de préférence choisi parmi un agent de charge, un solvant aqueux, un solvant organique, de préférence un alcool inférieur tel que l'éthanol, le propanediol, le butylène glycol, la glycérine ou l'isopropanol, un agent lipophile et leurs mélanges, et
- en option, de 0,1 à 30% d'un excipient supplémentaire acceptable sur le plan pharmaceutique ou cosmétique, de préférence choisi parmi un agent de vectorisation, un agent antioxydant, un agent conservateur, un agent stabilisant, un agent épaississant, un émulsifiant, un agent gélifiant hydrophile ou lipophile, un parfum, une huile minérale ou organique, et leurs combinaisons.
les pourcentages étant exprimés en poids par rapport au poids total de la composition cosmétique.

Comme mentionné ci-avant, l'extrait végétal est de préférence un extrait obtenu à partir d'une espèce de *Swertia* et ayant une teneur en swertiamarine d'au moins 90%, de préférence d'au moins 95%, en poids.

A titre d'agent de charge, on peut citer la silice et ses dérivés tels que le silicate de magnésium le talc, un amidon, un dérivé de l'amidon tel que la maltodextrine et leur mélange.

En tant qu'agent lipophile, on peut citer les acides gras, les esters d'acide gras tel que l'isopropyle palmitate, et/ou les alcools gras, de préférence en C₁₀-C₃₀.

En tant qu'agent émulsifiant ou de vectorisation, on peut citer les phospholipides tels que la phosphatidylcholine, la phosphatidylsérine, la phosphatidyléthanolamine, le phosphatidylglycérol, l'acide phosphatidique, le phosphatidylinositol, les lysophospholipides, leurs dérivés hydrogénés, et leurs mélanges.

La composition peut comprendre une phase aqueuse ou hydro-alcoolique obtenue à partir d'une eau minérale, d'une eau thermale ou d'une eau florale telle qu'une eau de camomille, de mauve, de bleuet ou d'hamamelis.

Les agents antioxydants comprennent, entre autres, le tocophérol et ses dérivés, la tocoquinone, HMR (hydroxy méthyl résinol), les polyphénols, les isoflavones, le propyl gallate, l'hydroxy anisol butylée (BHA), l'hydroxy toluène butylé (BHT) et leurs dérivés, le chlorhydrate d'histidine hydrochloride ou encore un extrait d'*Epilobium augustifolium.*

On peut citer à titre d'exemple de composés conservateurs ou d'agents antimicrobiens le 2-bromo-2-nitropropane-1,3-diol, le chlorure de behentrimonium, le chlorure de benzalkonium, l'alcool benzylique, butylparabène, le chlorure de cétrimonium, les dérivés de la chlorhexidine (digluconate, dihydrochlorure), la chlorphénésine, le climbazole, la diazolidinyle urée, la DMDM hydantoine, l'éthylparabène, le diiséthionate d'hexamidine, l'imidazolidinyl urée, le butylcarbamate d'iodopropynyle, l'isobutylparabène, l'isopropylparabène, l'isothiazolinone, le linalol, le benzoate de méthyle, la méthylchloroisothiazolinone, le méthyldibromoglutaronitrile, la méthylisothiazolinone, le méthylparabène, le o-cymén-5-ol, le phénoxyéthanol, l'olamine de piroctone, le polyaminopropyl biguanide, le propylparabène, le quaternium-15, le métabisulfite de sodium, le méthylparabène de sodium, le propylparabène de sodium, , un chlorure de stéaralkonium, le triclosan, la pyrithione de zinc, le caprylyl glycol, le caprate et caprylate de glycéryle, les acides organiques et leurs sels, de préférence de sodium ou de potassium, par exemple, l'acide sorbique et ses sels, l'acide benzoïque et ses sels, l'acide salicylique et ses sels, l'acide formique et ses sels, l'acide déhydroacétique et ses sels, l'acide lévulinique et ses sels, ou encore l'acide anisique (acide methoxybenzoïque) et ses sels.

La composition « précurseur » selon l'invention peut se présenter sous différentes formes. Il peut s'agir d'une composition solide, de préférence sous la forme d'une poudre, dans laquelle la swertiamarine peut être absorbée sur un agent de charge. Alternativement, la composition peut se présenter sous la forme d'une solution aqueuse, d'une solution hydroalcoolique ou d'une émulsion inverse (émulsion eau dans l'huile).

La composition « précurseur » selon l'invention est choisie parmi :
- une composition solide se présentant sous la forme d'une poudre dans laquelle la swertiamarine est absorbée sur un agent de charge, et
- une composition se présentant sous la forme d'une émulsion eau-dans-huile comprenant un agent lipophile de préférence choisi parmi les acides gras, les esters d'acides gras et/ou les alcools gras et un agent émulsifiant ou de vectorisation choisi parmi les phospholipides, leurs dérivés hydrogénés et leurs mélanges.

A titre d'exemple, l'agent de charge peut être une maltodextrine et/ou de la silice. A titre d'exemple supplémentaire, l'agent lipophile est le palmitate d'isopropyle et/ou l'agent vectorisation est un phospholipide ou un mélange de phospholipides, éventuellement hydrogénées, par exemple une lécithine hydrogénée ou non-hydrogénée.

A titre d'exemple spécifique, la composition « *précurseur »* selon l'invention peut être sous la forme d'une poudre comprenant:
- de 0,5% à 5% en poids d'un extrait végétal de préférence de *Swertia* telle que *Swertia Chirata,* enrichi en swertiamarine,
- de 90% à 99,5% en poids de maltodextrine, l'extrait végétal étant absorbé sur la maltodextrine.

A titre d'exemple supplémentaire, la composition « *précurseur »* selon l'invention peut être une émulsion eau-dans-huile comprenant:
- de 70% à 90% en poids de palmitate d'isopropyle,
- de 5% à 20% en poids de phospholipides, par exemple de lécithine
- de 0,5% à 5% en poids d'un extrait végétal de préférence de *Swertia* telle que *Swertia Chirata,* enrichi en swertiamarine, et
- de 1% à 8% en poids d'eau.

La présente invention a également pour objet l'utilisation de ladite composition « précurseur » pour la préparation d'une composition cosmétique ou pharmaceutique. Il va de soi que ladite composition cosmétique ou pharmaceutique finale est particulièrement adaptée pour la mise en œuvre de l'une quelconque des utilisations thérapeutiques ou cosmétiques selon l'invention, et peut présenter l'une quelconque des caractéristiques décrites ci-avant dans la partie intitulée *« Compositions contenant la swertiamarine selon l'invention ».*

La composition « précurseur » peut être présente dans la composition cosmétique ou pharmaceutique finale à hauteur de 0,001% à 50%, de préférence de 0,01% à 10% en poids par rapport au poids total de la composition cosmétique ou pharmaceutique finale.

Typiquement la composition cosmétique ou pharmaceutique peut être obtenue en mélangeant une composition « précurseur » selon l'invention avec un ou plusieurs excipients ou véhicules acceptables sur le plan pharmaceutique ou cosmétique.

Il va de soi que la présente invention a également pour objet un procédé de préparation d'une composition cosmétique ou pharmaceutique comprenant une étape de mélange de la composition « précurseur » selon l'invention avec un ou plusieurs excipients ou véhicules acceptables sur le plan pharmaceutique ou cosmétique.

### ▪ Autres utilisations de la swertiamarine

il est également décrit des utilisations *in vitro* de la swertiamarine, en particulier dans le domaine de la recherche ou dans le domaine médical. La swertiamarine peut être utilisée pour promouvoir la croissance d'explants de peau *in vitro.* Les explants de peau peuvent être utilisés en tant que greffe ou autogreffe pour le traitement d'une lésion cutanée, ou encore à des fins de recherche, par exemple pour l'évaluation d'agents actifs.

Il est aussi décrit un procédé de culture *in vitro* d'un explant de peau comprenant une étape de traitement de l'explant de peau par de la swertiamarine. La swertiamarine peut être typiquement ajoutée dans un milieu de culture approprié pour la culture ou le maintien d'un explant de peau ou être appliquée sur l'explant.

D'autres aspects et avantages de la présente invention apparaîtront à la lecture des exemples qui suivent, qui doivent être considérés comme illustratifs et en aucun cas comme limitatifs.

### EXEMPLES

### EXEMPLE 1 : La swertiamarine stimule la production du facteur de croissance de kératinocyte (Keratinocyte Growth Factor - KGF) par les adipocytes.

Le but de cette étude était de quantifier le facteur de croissance de kératinocyte (Keratinocyte Growth Factor - KGF) dans le milieu de culture d'adipocytes humains mis en culture avec et sans swertiamarine.

### Protocole

Une solution stock à 10% en poids de swertiamarine dans le DMSO a été préparée. Cette solution a été ensuite diluée dans le milieu de culture de manière à obtenir une concentration finale de 0,0075% en poids de swertiamarine.

Une culture d'adipocytes humains normaux, obtenue à partir d'adipocytes prélevés par abdominoplastie chez une femme de 34 ans, a été incubée pendant 15 heures avec le milieu de culture contenant la swertiamarine. A titre de témoin négatif, on a utilisé une culture d'adipocytes incubée pendant 15 heures avec un milieu de culture exempt de swertiamarine mais contenant la même quantité de DMSO.

A l'issue de l'incubation, la quantité de KGF a été quantifiée dans chaque milieu de culture par un test ELISA. La signification statistique des différences entre les groupes « contrôles » et les « groupes test » a été évaluée par une analyse de la variance (One way Anova) suivi par le test Holm Sida (p*<0,05).

### Résultats

L'analyse statistique des résultats obtenus pour les différents groupes d'expérience montrent une augmentation significative de la quantité de KGF dans les milieux de culture des adipocytes incubés en présence de swertiamarine par rapport aux groupes témoins. Une quantité de 0,0075% en poids de swertiamarine induit une augmentation de la quantité moyenne de KGF dans le milieu de culture d'environ 16,4%.

### EXEMPLE 2 : La swertiamarine stimule la prolifération des kératinocytes.

### ▪ Modèle 1 : Test dit « scratch assay »

Le but de cette étude était d'évaluer l'effet de la swertiamarine sur un modèle de culture monocouche de kératinocytes humains.

### Protocole

Des kératinocytes normaux prélevées chirurgicalement chez une femme âgée de 45 ans ont été mis en culture de manière à obtenir une culture monocouche de kératinocytes.

Lorsque la confluence a été atteinte, une lésion (« scratch ») a été effectuée dans chaque culture cellulaire de manière à enlever localement une quantité équivalente de kératinocytes.

Chaque culture de kératinocytes a été ensuite incubée à 37°C en atmosphère humide et en présence de 5% de CO₂, en présence d'un milieu de culture adipocytaire conditionné par de la swertiamarine (0,0025% ; 0,005% ; 0,0075% en poids), pendant 72 heures. La swertiamarine a été introduite dans le milieu de culture adipocytaire par dilution d'une solution stock à 10% dans le DMSO. La quantité finale de DMSO dans les milieux de culture est de 0,15%. A titre de contrôle, on a utilisé une culture de kératinocyte incubée dans les mêmes conditions en présence d'un milieu de culture comprenant des adipocytes humains normaux en suspension exempt de swertiamarine mais contenant 0.15% de DMSO (expérience Contrôle DMSO). A des fins de validation, une culture de kératinocytes a été incubée en présence du facteur de croissance TGF-β (10 ng/ml) dans les mêmes conditions (Contrôle TGF-β).

La surface correspondant à la « lésion » (c'est-à-dire à la zone où les kératinocytes ont été retirés) a été mesurée avant (t= 0) et après incubation pour chaque culture cellulaire à partir de clichés obtenus par microscopie optique en utilisant le logiciel Image J.

Les résultats ont été exprimés en pourcentage de surface recolonisée par rapport à la surface observée à t=0.

La signification statistique des différences obtenues entre les groupes « Contôle DMSO » et les groupes « Contrôles TGF-β » a été évaluée en utilisant le test-t (*** : p<0,001). La signification statistique des différences obtenues entre les groupes « Contrôle DMSO » et les groupes test « SWT » a été analysé par une analyse de la variance (One way Anova) suivi par le test Holm Sida (p*<0,05).

### Résultats

Les résultats obtenus sont illustrés à la Figure 1 qui montre le pourcentage moyen de recolonisation obtenu après incubation pour chaque groupe d'expériences.

De manière notable, le produit de référence, à savoir le TGF-β, a significativement induit la recolonisation cellulaire après 72 heures de stimulation (+124,1% ; p<0.001). Ce résultat était attendu et a permis de valider l'ensemble des expériences.

Par ailleurs, le milieu de culture adipocytaire conditionné par de la swertiamarine a stimulé de manière significative la prolifération des kératinocytes et la recolonisation. La swertiamarine a donc induit fortement le processus de cicatrisation comparativement aux expériences « Contrôle DMSO ». Cet effet d'induction de la cicatrisation est observable à toutes les concentrations testées, y compris à 0,0025% de swertiamarine où on observe une augmentation de +64,6% de la surface recolonisée par les kératinocytes par rapport au groupe « Contrôle DMSO ».

La swertiamarine stimule donc la prolifération des kératinocytes et ainsi la régénération de l'épiderme.

### ▪ Modèle 2 : Explant de peau

Le but de cette étude était d'évaluer l'effet de la swertiamarine sur des explants de peau humain avec et sans tissu adipeux.

### Protocole

Des échantillons de peau ont été prélevés chirurgicalement chez une femme caucasienne au cours d'une abdominoplastie. 21 explants de peau de 10 mm de diamètre ont été préparés à partir de ces échantillons, parmi lesquels 12 sont des explants de peau sans tissu adipeux (explant épiderme + derme) et 9 sont des explants de peau entière (épiderme + derme + hypoderme). Les explants sont maintenus en vie dans un milieu BEM à 37°C en présence d'une atmosphère humide à 5% de CO₂. Les explants de peau ont été répartis en trois groupes :
- Groupe 1 : une crème contenant de la swertiamarine (SWT) à hauteur de 0,025 % a été appliquée aux jours 0, 1, 2, 3, 6 et 7 de l'incubation, à la surface des explants de peau à l'aide d'une spatule (1 µl le matin et 1 µl le soir),
- Groupe 2 : une crème placebo (exempte de swertiamarine) a été appliquée aux jours 0, 1, 2, 3, 6 et 7 de l'incubation, à la surface des explants de peau à l'aide d'une spatule (1 µl le matin et 1 µl le soir) (1 µl le matin et 1 µl le soir),
- Groupe 3 : ce groupe témoin a été maintenu en vie sans traitement (sans application de crème placebo ou de crème à base de swertiamarine)

Au jour 9, les explants de peau sont récupérés, fixés chimiquement dans une solution de formol pendant 24 heures, déshydratés, imprégnés de paraffine puis infiltrés par de la résine selon le protocole SOP H-53. Des coupes fines sont ensuite réalisées dans chaque explant et observées par microscopie optique après coloration.

### Résultats

Les explants de peau entière traités avec de la swertiamarine présentent, au bout de 9 jours, entre 10 et 12 couches cellulaires d'épiderme contre seulement 4-5 pour les mêmes explants de peau non traités, et 4-5 pour ceux traités avec le placebo. La swertiamarine a donc induit un épaississement significatif de l'épiderme. De plus, les explants de peau traités par la swertiamarine ne présentent pas d'altération morphologique notable. La swertiamarine a également été capable d'induire un épaississement de l'épiderme chez des explants de peau dépourvus de tissu adipeux (épaisseur de 6 à 7 couches cellulaires), ce qui montre que la swertiamarine exerce également un effet direct sur les kératinocytes. Ces résultats sont illustrés à la Figure 3.

Cette expérience illustre de nouveau la capacité de la swertiamarine à induire la régénération de l'épiderme et la prolifération des kératinocytes.

### EXEMPLE 3 : La swertiamarine stimule la synthèse de fibronectine par les fibroblastes de la peau

La fibronectine est une protéine d'adhésion jouant un rôle central dans l'ancrage des cellules dans la matrice extracellulaire. Elle est également impliquée dans la modulation des fonctions cellulaires. La fibronectine est essentiellement localisée dans le derme et au niveau de la jonction entre le derme et l'épiderme. Cette protéine est sensible au clivage protéolytique, un phénomène qui est accentué au cours du vieillissement et qui a pour conséquence d'affecter l'intégrité du derme en diminuant la quantité de fibronectine et en augmentant la quantité de protéines dénaturées.

Le but de cette étude était d'identifier l'effet de la swertiamarine sur la production de fibronectine par les fibroblastes du derme.

### Protocole

Le test mis en œuvre était basé sur la synthèse de fibronectine induite by TGF-β par des cellules de fibroblastes humains normaux du derme (NHDF) en culture monocouche. La culture a été realisée en utilisant en tant que milieu de culture du DMEM (Eurobio, CMODME70-08) contenant 10 % de sérum fétal de veau (Eurobio, CVFSV00-0U), 1 % d'antibiotiques (penicilline/streptomycine, Eurobio, CABPES01-OU) and 1 % L-glutamine (Eurobio, CSTGLUOO-OU), à une température de 37 ° C sous 5 % de CO₂ and 95 % d'humidité.

Les cellules ont été ensuite ensemencées, en présence de DMEM complet, dans des microplaques à raison de 2.5^{∗}10⁴ cellules par puits puis incubées à une température de 37 ° C sous 5 % de CO₂ and 95 % d'humidité. Le milieu de culture a été remplacé au bout de 24 heures pour amener les cellules à un état de quiescence.

Ensuite, les cellules ont été incubées en présence de TGF-β ou de swertiamarine pendant 24 h ou 48h. Les surnageants cellulaires ont été ensuite collectés afin de quantifier la fibronectine par un test ELISA (BMS028, Ebiosciences). Le taux de viabilité cellulaire a été également déterminé par le test classique MTT. A titre de contrôle, des cellules ont été incubées dans les mêmes conditions mais en l'absence de TGF-β et de swertiamarine (contrôle).

Les résultats ont été exprimés en pourcentage de quantité de fibronectine produite par mL par rapport à la quantité de fibronectine produite pour le groupe d'expériences de contrôle.

### Résultats

Les résultats obtenus sont illustrés à la Figure 2 qui montre le pourcentage moyen de fibronectine produite dans les groupes d'expérience. Le pourcentage est exprimé par rapport à la quantité de fibronectine produite pour l'expérience contrôle.

On observe que le produit de référence, à savoir le TGF-β, a significativement induit la production de fibronectine (+64%). Ce résultat était attendu et a permis de valider l'ensemble des expériences.

De manière remarquable, la swertiamarine a induit très fortement la synthèse de fibronectine par rapport au contrôle, et ceci à toutes les concentrations testées. Cet effet est généralement plus élevé que celui observé avec le TGF-β. A titre d'illustration, une concentration de 0.005% de swertiamarine a induit une augmentation de plus de 171% de la quantité de fibronectine par rapport aux expériences contrôles.

La swertiamine est donc également capable de restaurer ou d'améliorer l'intégrité du derme en induisant la synthèse de fibronectine.

### EXEMPLE 4 : Exemples de compositions « précurseurs » selon l'invention

Les compositions précurseur suivantes ont été préparées à partir d'un extrait de *Swertia Chirata* présentant une teneur d'au moins 95% en poids de swertiamarine (ci-après extrait SWT).

### ▪ Composition A (sous forme de poudre) :

| **Composition A (% en poids)** | |
|---|---|
| Extrait SWT | 1,25% |
| Silice | 2,00% |
| Maltodextrine | 96,75% |

### ▪ Compositions B et C (Compositions sous forme de solution hydroalcoolique)

| **Composition B (% en poids)** | |
|---|---|
| Extrait SWT | 1,25% |
| Propanediol | 70,00% |
| Eau | 28,75% |

| **Composition C (% en poids)** | |
|---|---|
| Extrait SWT | 1,25% |
| Glycérine | 70,00% |
| Eau | 28,75% |

### ▪ Composition D : Composition sous forme d'émulsion inverse

| **Composition D (% en poids)** | |
|---|---|
| Phospholipides | 15,8% |
| Isopropyl palmitate | 80,7% |
| Eau | 2,25% |
| Extrait SWT | 1,25% |

### ▪ Composition E : Composition sous forme de poudre

| **Composition E (% en poids)** | |
|---|---|
| Extrait SWT | 1,25% |
| Maltodextrine | 98,75% |

### EXEMPLE 5 : Exemples de compositions cosmétiques selon l'invention

A titre d'exemples, les produits cosmétiques suivants peuvent être préparés. Ces produits cosmétiques intègrent une des compositions « précurseur » décrites ci-avant.

### ▪ Gel huileux anti-vergetures

L'extrait de swertiamarine y est présent à titre d'agent lissant de la peau. Ce gel huileux peut être appliqué sur la peau à traiter, en particulier au niveau des seins, du ventre et des cuisses, typiquement une à deux fois par jour.

| Phase | Ingrédient | Nom INCI | % en poids* |
|---|---|---|---|
| A | Emulmetik™ 950 | Lécithine hydrogénée | 1,00 |
| B | Glycérine | Glycérine | 49,00 |
| | Butylène Glycol | Butylène Glycol | 19,00 |
| C | Mirasil PTM | Phényl trimethicone | 28,00 |
| D | **Composition précurseur D** | Phospholipides (et) isopropylpalmitate (et) eau (et) extrait de *Swertia Chirata* | 2,00 |
| E | Sveltessence™ | Glycérine (et) eau (et) extrait de graines de *Nephelium Longana* | 1,00 |

| | | | |
|---|---|---|---|
| * par rapport au poids total du gel | | | |

### ▪ Baume réparateur pour lèvres

L'extrait de swertiamarine y est présent en tant qu'agent régénérateur de l'épiderme pour le traitement et la prévention des gerçures. Le baume à lèvres peut être appliqué sur les lèvres typiquement une à deux fois par jour.

| Phase | Ingrédient (dénomination INCI) | % en poids* |
|---|---|---|
| **A** | Cire de Euphorbia | 9 |
| | Cellulose microcrystalline | 4,40 |
| | Ozokérite | 2,50 |
| | Cire de *Copernicia cerifera* | 2,50 |
| | Octyldodécanol | 10 |
| | Cocoglycérides | 5,00 |
| | Dicaprylyl carbonate | 4,00 |
| | Propylheptylcaprylate | 6,00 |
| | Polyglyceryl-2 triisostéarate | 26,35 |
| | **Composition précurseur D (extrait SWT)** | 2,50 |
| **B** | CI15850 | 1,80 |
| | Ci77891 | 4,50 |
| | Ci77499 | 0,15 |
| | Lécithine (et) polyglycéryl-3-palmitate | 3,70 |
| | Lécithine | 14,80 |
| **C** | Tocopherol (et) huile de grains d'*Helianthus annuus* | 0,50 |
| | Parfum | 0,50 |
| **D** | Eau (et) glycérine (et) extrait de graine de *Linum usitatissimum* | 1,30 |
| | Eau | 1,00 |
| | Propanediol | 1,00 |
| | Lécithine hydrogénée (et) alcools C₁₂-C₁₆ (et) acide palmitique | 0,80 |
| | Parfum | 0.20 |

| | | |
|---|---|---|
| * par rapport au poids total du baume | | |

### ▪ Crème de jour antiride

L'extrait de swertiamarine y est présent en tant qu'agent antiride, c'est-à-dire en tant qu'agent pour prévenir ou atténuer les rides et/ou les ridules. Cette crème est typiquement appliquée sur le visage, le matin de préférence, sur une peau propre et avant le maquillage.

| Phase | Ingrédient | Dénomination INCI | % en poids* |
|---|---|---|---|
| A | Eau déionisée | eau | 52,70 |
| | FDC Red 4 Solution 0,1% | eau (et) CI 14700 | 0,20 |
| B | Veegum HS | Silicate de Magnésium et d'Aluminum | 0,50 |
| | Glycérine | Glycérine | 5,00 |
| | Natrosol 250 M Pharm | Hydroxyéthylcellulose | 0,30 |
| C | Chlorphénésine | Chlorphénésine | 0,30 |
| | Dermofeel™ PA-3 | Phytate de sodium (et) eau | 0,10 |
| | Biophilic™ H | Lécithine hydrogénée (et) alcools C₁₂-C₁₆ (et) acide palmitique | 4,00 |
| D | Cegesoft PS 6 | Huile végétale | 3,00 |
| | Cetiol C5 | Coco-Capry late | 3,00 |
| | Lipex Shea | Beurre de *Butyrospermum Parkii* | 5,00 |
| | Dc 200,5Cs | (Shea) | 3,50 |
| | Dermofeel™ BGC | Diméthicone | 7,00 |
| | Dermofeel™ GSC | Butylène Glycol Dicaprylate/ Dicaprate | 1,00 |
| | Stearine | Glycéryl Stéarate Citrate | 1,00 |
| | Dermofeel™ Toco 70 | Acide stéarique | 0,20 |
| | Non Gmo | Tocophérol (et) huile de graine de *Helianthus Annuus* | |
| E | Lanablue® | Sorbitol (et) Eau (et) extrait d'algue | 1,00 |
| | Mamaku Vital Essence Nature PF | Eau (et) Glycérine (et) extrait de feuille de *Cyathea Medullaris* | 2,00 |
| | Sculptessence™ | Eau (et) Glycérine (et) extrait de graine *de Linum usitatissimum* | 5,00 |
| | **Composition précurseur C** | Glycérine (et) eau (et) extrait de *Swertia Chirata* | 4,00 |
| F | Dermosoft™ 1388 | Fragrance | 3,00 |
| G | SJ Touch 1 | Polyméthyl Methacrylate | 2,00 |
| H | Elegance 4042 | Fragrance | 0,20 |

| | | | |
|---|---|---|---|
| * par rapport au poids total de la crème | | | |

### ▪ Crème anti-rougeur

| Phase | Ingrédient | Nom INCI | % en poids* |
|---|---|---|---|
| **A** | Eau déionisée | Eau | 66,1 |
| | Glycérine | Glycérine | 4 |
| | Dermosoft™ GMCY | Glyceryl Caprylate | 0,5 |
| | Satiaxane CX 91 | Gamme de xanthane | 0,5 |
| | Biophilic™ H | Lécithine hydrogénée (et) alcools C₁₂-C₁₆ (et) acide palmitique | 4 |
| **B** | Huile de tournesol | Huile de graine d'*Helianthus Annuus* | 5 |
| | Huile de noisette | Huile de *Corylus Avellana* | 5 |
| | Cire d'abeille | Cire d'abeille | 4 |
| | Dermofeel™ Toco 70 Non Gmo | Tocophérol (et) huile de graine de *Helianthus Annuus* | 0,2 |
| | Shea Butter | Butyrospermum Parkii (Shea) Butter | 3 |
| **C** | Deionised Water | Water | 5 |
| | **Composition précurseur A** | Maltodextrine (et) silice (et) extrait de *swertia chirata* | 2 |
| **D** | Sorbate de potassium | Sorbate de potassium | 0,3 |
| **E** | Relax 2020/2 | Fragrance | 0,4 |

| | | | |
|---|---|---|---|
| * par rapport au poids total de la crème | | | |

### EXEMPLE 6 : la swertiamarine atténue de manière visible les rides faciales

L'effet *in vivo* de la swertiamarine sur les rides faciales a été évalué au travers de 4 essais cliniques. Le but de ces essais était de démontrer l'effet de l'application quotidienne d'une crème contenant 2% de la composition D (soit environ 0,024% de swertiamarine) sur la profondeur des rides et le relief de la peau.

Cette crème, dénommée ci-après « crème SWT », présente la composition suivante :

| **Phase** | **Ingrédient** | **Nom INCI** | **% en poids** |
|---|---|---|---|
| **A** | Eau déionisée | Eau | 69,95 |
| | Dermofeel™ PA3 | Sodium phytate (et) eau (et) alcool | 0,10 |
| **B** | Glycerin | Glycerine | 2,00 |
| | Satiaxane CX911 | Gomme de xanthane | 0.25 |
| **C** | Heliofeel | Glycéryl Stéarate Citrate (et) Polyglycéryl-3 Stéarate (et) Lécithine hydrogénée | 4,00 |
| | **Composition précurseur D** | Phospholipides et isopropyl palmitate et eau et extrait de S. Chirata | 2,00 |
| | LIPEX® 102 | Beurre de Butyrospermum Parkii | 7,00 |
| | Sweet Almond Oil | Huile d'amande douce | 8,00 |
| | Lanette® 22 | Behenyl alcohol | 2,50 |
| | Vitapherole E1000 | Tocophérol (et) huile de graine d'*Helianthus annus* | 0,20 |
| **D** | Dermosoft™ 1388 | Parfum | 4,00 |
| | | | 100,00 |

La crème « Placebo », utilisée dans les essais cliniques, se distingue de la crème « SWT » en ce que l'extrait de *S*. *chirata* dans la composition précurseur D a été remplacé par de l'eau.

Les études se sont intéressées à différents types de rides faciales, en particulier aux rides verticales telles que la ride du bélier, les rides de la région des sillons nasogéniens et les rides du contour des lèvres.

### - Rides verticales au niveau du bas du visage

L'effet de la swertiamarine sur les rides verticales du bas du visage a été caractérisé par deux essais cliniques « *swertiamarine vs. placebo ».*

### Essai clinique n°1 :

Le premier essai clinique avait pour but d'évaluer l'effet antiride et lissant de la swertiamarine. 16 femmes volontaires, en bonne santé, âgées de 45 à 65 ans ont été enrôlées. Ces volontaires ont reçu une « crème SWT » contenant 2% de la composition D (soit environ 0,024% de swertiamarine) et la «crème placebo» identique à la crème SWT mais dépourvue d'extrait de *Swertia chirata.* Chaque volontaire a appliqué sur une première moitié de son visage la «crème placebo » et sur la seconde moitié de son visage la «crème SWT», à raison d'une fois le matin et une fois le soir pendant 28 jours. L'effet antiride de la swertiamarine a été évalué au niveau des parties basses du visage à J=0, 7 et 28 du traitement grâce au système d'imagerie faciale Visia-CR® (Canfield scientific) en utilisant les filtres « rides » et « textures ». L'analyse par imagerie a été focalisée sur les rides et la texture de la peau. Le traitement statistique des données a été réalisé par un test de student sur des données appariées (paired student t-test). Le traitement a consisté à comparer les valeurs obtenues pour les zones du visage traitées avec la crème SWT et les valeurs obtenues pour la crème placebo, aux différents temps (Jt) de traitement, en prenant comme référence les valeurs à J=0 (c'est-à-dire que l'on a comparé les paires de données (J0, Jt) pour la crème SWT *versus* les paires de données (J0, Jt) pour la crème placebo).

Les résultats de l'étude ont montré qu'une diminution significative des rides (-16%, p<0,05) et de la rugosité de la peau (-9%, p<0,05) au bout de 28 jours sur les zones du visage traitées avec la crème « SWT » par rapport aux zones du visage traitées avec la « crème placebo ». De manière notable, l'amélioration de l'aspect de la peau a été constatée dès le septième jour de traitement. Chez certains sujets, on a pu observer une réduction des rides de 53% et une réduction de la rugosité de la peau de 42% à J=7 par rapport à J=0. A titre d'exemple, la Figure 4 montre les photographies (avec le filtre « ride ») de la partie basse du visage d'une volontaire âgée de 51 ans à J = 0 (D0) et après 7 jours de traitement avec la crème SWT (D7). On note sur la photographie à J7 (D7), une diminution nette de la surface des rides au niveau de la joue et une atténuation nette de la profondeur du sillon nasogénien.

### Essai clinique n°2 :

Le but de cet essai était de confirmer l'effet antiride et lissant de la swertiamarine sur des rides verticales. 17 femmes volontaires en bonne santé, âgées entre 45 et 65 ans, présentant des rides verticales dans la partie basse de leur visage ont été enrôlées pour les besoins de cet étude. Le protocole suivi a été identique à celui de l'essai clinique n°1. L'effet antiride a été évalué aux jours J=0, 7, 14 et 28 sur les parties basses du visage par une analyse 3D de l'empreinte de la peau afin de suivre l'évolution du volume des rides au cours du temps par profilométrie par projection de franges (fringe projection) sur empreinte de peau. L'analyse statistique a été réalisée par un test de student sur données appariées.

Les résultats obtenus confirment l'effet antiride observé lors du premier essai clinique : une nette diminution du volume des rides verticales pour les zones du visage traitées avec la « crème SWT » a été constatée dès le 7^{ème} jour de traitement par rapport aux zones du visage traitées avec la crème placebo. L'application de la crème SWT a permis de limiter de manière notable le volume des rides verticales. Cet effet est illustré par la Figure 5 qui montre le pourcentage de variation du volume des rides par rapport à la valeur de référence mesurée à J=0, pour les peaux traitées par la crème placebo (Placebo) et les peaux traitées avec la crème « SWT ». On observe une augmentation du volume des rides pour les peaux traitées avec la crème placebo. En revanche, l'application biquotidienne de la crème « SWT» permet de diminuer le volume des rides au cours du temps, illustrant l'effet antiride de la swertiamarine.

### - Rides du contour des lèvres

2 essais cliniques supplémentaires ont été réalisés par un dermatologue afin de caractériser l'effet de la swertiamarine sur les rides du contour des lèvres.

### Essai clinique n°3 :

Cet essai a enrôlé 10 femmes volontaires en bonne santé, fumeuses, âgées de 45 à 65 ans, présentant des rides marquées au niveau des lèvres (typiques d'une consommation régulière de tabac). Les volontaires ont reçu une « crème SWT » contenant 2% de la composition D (soit environ 0,024% de swertiamarine) et une « crème placebo » avec pour consigne d'appliquer une première crème sur la première moitié de leur visage, et la seconde crème sur l'autre moitié de leur visage, le matin et le soir, pendant 28 jours. Aux jours de traitement J = 0, 7 et 28, une analyse de la variation du relief cutané sur chaque moitié du visage a été réalisée en utilisant le système Primos® 3D Pico (basée sur la technique dite « Fringe projection »). Cette analyse a été focalisée sur les rides des lèvres et a eu pour but de déterminer la rugosité moyenne (Ra). Ra correspond au rapport entre la surface intégrée autour de la valeur moyenne sur la longueur du profile. Une diminution de Ra caractérise un effet lissant. L'analyse statistique a été effectuée par le test de student sur des données appariées (paired student t-test).

L'analyse de la variation du relief cutané a mis en évidence une diminution significative de la rugosité moyenne (Ra) (-19%, p<0.05) au bout de 28 jours sur la zone du contour des lèvres traitées avec la « crème SWT » par rapport aux zones traitées avec la « crème Placebo ». A titre d'exemple, la Figure 6 montre des macrophotographies du contour des lèvres d'une volontaire âgée de 64 ans à J = 0 (D0), J=7 (D7) et J=28 (D28) du traitement avec la crème SWT. On observe une nette diminution du relief des rides au niveau du contour supérieur de la lèvre sur les macrophotographies D7 et D28. La peau paraît plus lisse.

### Essai clinique n°4 :

Cet essai a enrôlé 10 femmes volontaires en bonne santé, fumeuses, âgées de 45 à 65 ans, présentant des rides marquées au niveau des lèvres. Chaque femme enrôlée dans l'essai clinique a appliqué uniformément la crème « SWT » sur l'ensemble de son visage, pendant 28 jours, à raison de deux fois par jour (le matin et le soir).

Un test de migration de rouge à lèvres a été réalisé à J=0 et J=28 du protocole. A cette fin, un rouge à lèvres a été appliqué sur l'ensemble des lèvres de chaque volontaire à J= 0 et J=28. Deux heures après l'application du rouge à lèvres, des photographies des lèvres ont été réalisées avec une lumière polarisée croisée et des Prolite® flashs en utilisant un appareil photographique D7100 Nikon avec un 60 mm Nikon. Les prises ont été réalisées avec une vitesse d'obstruction de 1/125 seconde et un diaphragme sur f22. Le positionnement des sujets a été standardisé pour respecter la proportion 1/3.

Les photographies ont été visualisées sur ordinateur. Le score de migration a été déterminé par le technicien en charge de l'étude et un technicien indépendant à J=0 et à J=28 selon l'échelle suivante : 0 : pas de migration, 1 : migration légère, 2 : migration modérée, 3 : migration importante, 4 : migration très importante.

Cet essai clinique a permis d'observer une migration du rouge à lèvres en moyenne 2 fois moins importante à J =28 qu'à J =0 sur l'ensemble des femmes enrôlées.

Chez certaines femmes, l'application de la crème SWT deux fois par jour, pendant 28 jours, a permis de diminuer par un facteur 7 le score de migration du rouge à lèvres par rapport à la donnée de migration déterminée à J=0.

A titre d'exemple, la Figure 6 montre la photographie du contour des lèvres d'une volontaire âgée de 50 ans à J = 0 (D0), et à J=28 (D28) du traitement avec la crème SWT. La migration du rouge à lèvres est significativement plus importante à J=0 qu'à J =28.

Les résultats de cette étude soulignent, une fois encore, la capacité de la swertiamarine à atténuer les rides.

## Revendications

1. Utilisation cosmétique non-thérapeutique de la swertiamarine ou d'un extrait végétal enrichi en swertiamarine en tant qu'agent stimulant la formation ou la régénération de l'épiderme et/ou en tant qu'agent stimulant le métabolisme du derme pour traiter ou prévenir une altération non-pathologique de la peau ou pour améliorer l'aspect de la peau, dans laquelle la swertiamarine ou ledit extrait est présent(e) à titre d'agent actif dans une composition destinée à l'administration par voie topique et ladite composition comprend de 0,001% à 5% en poids de swertiamarine.

2. Utilisation cosmétique non-thérapeutique de la swertiamarine ou d'un extrait végétal enrichi en swertiamarine selon la revendication 1, dans laquelle ledit extrait végétal est un extrait obtenu à partir d'une espèce de *Swertia,* de préférence *Swertia chirata* ou *Swertia milensis,* et ledit extrait comprend au moins 80% en poids de swertiamarine.

3. Utilisation cosmétique non-thérapeutique de la swertiamarine ou d'un extrait végétal selon l'une quelconque des revendications 1 à 2, ladite swertiamarine ou ledit extrait végétal étant utilisé(e) à titre d'agent anti-âge, d'agent antiride, d'agent unificateur du teint, d'agent anti-rougeur ou d'agent pour lisser la peau.

4. Utilisation cosmétique non-thérapeutique de la swertiamarine ou d'un extrait végétal selon la revendication 1 ou 2, dans laquelle ladite composition est destinée
- à prévenir, ou traiter un signe du vieillissement cutané,
- à prévenir, ou traiter les vergetures,
- à rendre moins visible des micro-vaisseaux de surface, et/ou
- à prévenir, traiter ou atténuer une rougeur cutanée.

5. Utilisation cosmétique non-thérapeutique de la swertiamarine ou d'un extrait végétal selon la revendication 4, dans laquelle le signe du vieillissement cutané est choisi parmi le groupe constitué par un amincissement de la peau, en particulier de l'épiderme, l'apparition d'un microrelief, l'apparition de ridules et/ou de rides sur la peau, y compris au niveau des lèvres et des paupières, un flétrissement ou un affaissement de la peau, une perte d'éclat de la peau, les cernes, un teint brouillé, une perte de densité de la peau, une perte de fermeté de la peau, une perte de tonicité de la peau, une perte d'élasticité de la peau, une altération de l'aspect lisse de la peau, et/ou une augmentation de la rugosité de la peau.

6. Utilisation cosmétique non-thérapeutique de la swertiamarine ou d'un extrait végétal selon la revendication 4, dans laquelle la composition est destinée à traiter ou à prévenir une rougeur transitoire ou permanente, de préférence chez une peau choisie parmi le groupe constitué par une peau à tendance couperosique, une peau à tendance érythrosique et une peau à tendance érythro-couperosique.

7. Utilisation cosmétique non-thérapeutique de la swertiamarine ou d'un extrait végétal selon l'une quelconque des revendications 4 à 6, dans laquelle la swertiamarine représente entre 0,005% et 0,5% en poids du poids total de ladite composition cosmétique.

8. Utilisation cosmétique non-thérapeutique de la swertiamarine ou d'un extrait végétal selon l'une quelconque des revendications 4 à 7, la composition comprenant en outre, au moins un agent cosmétique additionnel, de préférence choisi dans le groupe constitué par les vitamines, les filtres et écrans solaires, les agents anti-âges, ou antirides, les antioxydants, les agents liftants, les agents raffermissants, les agents anti-tâches, les agents anti-rougeurs, les agents amincissants, les agents drainants, les agents hydratants, les agents apaisants, les agents gommants ou exfoliants, les agents matifiants, les agents séborégulateurs, les actifs éclaircissants, les actifs auto-bronzants, les accélérateurs de bronzage et leurs combinaisons.

9. Utilisation cosmétique non-thérapeutique de la swertiamarine ou d'un extrait végétal selon l'une quelconque des revendications 4 à 8, dans laquelle la composition est choisie parmi le groupe constitué par les solutions aqueuses, les solutions hydroalcooliques, les émulsions huile-dans-eau (H/E) ou eau-dans huile (E/H) ou multiple (triple : E/H/E ou H/E/H), les nanoémulsions, en particulier des nanoémulsions H/E, dont la taille des gouttes est inférieure à 100nm, les gels aqueux, ou les dispersions d'une phase grasse dans une phase aqueuse à l'aide de sphérules, les suspensions, de préférence en milieux aqueux ou hydroalcoolique, les suspensions de liposomes, les poudres, les lotions, laits, les crèmes, les onguents, les gels, les mousses, et les pommades.

10. Utilisation cosmétique non thérapeutique de la swertiamarine selon l'une quelconque des revendications 1à 9, dans laquelle la swertiamarine est utilisée sous la forme d'un extrait de *Swertia Chirata.*

11. Swertiamarine ou extrait végétal enrichi en swertiamarine pour une utilisation en tant qu'agent cicatrisant ou en tant qu'agent régénérateur de l'épiderme, dans le traitement ou la prévention d'une lésion de la peau ou d'une muqueuse causée par l'exposition de la peau ou de la muqueuse à une agression climatique, à un produit chimique, à un agent thérapeutique, à une radiothérapie, au laser ou à un procédé cosmétique, dans laquelle la swertiamarine ou l'extrait est présent(e) à titre d'agent actif dans une composition destinée à l'administration par voie topique et ladite composition comprend de 0,001% à 5% en poids de swertiamarine.

12. Swertiamarine ou extrait végétal enrichi en swertiamarine pour son utilisation selon la revendication 11 dans laquelle la lésion de la peau ou de la muqueuse est causée par une exposition de la peau ou de la muqueuse à une agression choisie dans le groupe constitué par une exposition au vent, une exposition aux variations de température, une exposition à un détergent, une exposition à un agent anti-cancéreux, une exposition à un agent anti-acnéique, une exposition à la radiothérapie, une exposition au laser, une exposition au peeling et une exposition à une dermo-abrasion.

13. Swertiamarine ou extrait végétal enrichi en swertiamarine pour son utilisation selon la revendication 11 ou 12, dans laquelle la lésion de la peau ou de la muqueuse est choisie parmi une coupure, une brûlure, une irritation, une ampoule, une cloque, des fissures, des micro-plaies, une gerçure, une crevasse et des fendillements.

14. Swertiamarine ou extrait végétal enrichi swertiamarine pour une utilisation selon l'une des revendications 11 à 13 dans laquelle la swertiamarine ou ledit extrait est administré en combinaison avec un autre principe actif, de préférence choisi parmi le groupe constitué par les agents apaisants, les agents hydratants, les agents anti-inflammatoires, les agents antioxydants, les agents cicatrisants, les agents désinfectants, les agents antimicrobiens et leurs combinaisons.

15. Composition pharmaceutique ou cosmétique pour une utilisation dans le traitement d'une lésion de la peau ou d'une muqueuse causée par l'exposition de la peau ou de la muqueuse à une agression climatique, à un produit chimique, à un agent thérapeutique, à une radiothérapie, au laser ou à un procédé cosmétique, ladite composition comprenant de 0,001% à 5% en poids de swertiamarine en tant qu'agent cicatrisant ou agent régénérateur de l'épiderme.

16. Extrait végétal enrichi en swertiamarine pour une utilisation selon l'une des revendications 11 à 12, où ledit extrait végétal est un extrait obtenu à partir d'une espèce de *Swertia,* de préférence *Swertia chirata* ou *Swertia milensis,* et qui comprend au moins 80% en poids de swertiamarine.

17. Composition précurseur destinée à être utilisée dans la préparation d'une composition pharmaceutique ou cosmétique, comprenant :
- de 0,1 à 20% de swertiamarine, ladite swertiamarine étant de préférence intégrée sous la forme d'un extrait végétal enrichi en swertiamarine comprenant de préférence au moins 80% en poids de swertiamarine, notamment un extrait obtenu à partir d'une espèce de *Swertia* telle que *Swertia chirata* ou *Swertia milensis,*
- de 50% à 99,9 % d'un véhicule, de préférence choisi parmi un agent de charge, un solvant aqueux, un solvant organique, un agent lipophile et leurs mélanges, et
- en option, de 0,1 à 30% d'un excipient supplémentaire acceptable sur le plan pharmaceutique ou cosmétique, de préférence choisi parmi un agent de vectorisation, un agent antioxydant, un agent conservateur, un agent stabilisant, un agent épaississant, un émulsifiant, un agent gélifiant hydrophile ou lipophile, un parfum, une huile minérale ou organique, et leurs combinaisons,
les pourcentages étant exprimés en poids par rapport au poids total de la composition, ladite composition étant choisie parmi :
- une composition solide se présentant sous la forme d'une poudre dans laquelle la swertiamarine est absorbée sur un agent de charge, et
- une composition se présentant sous la forme d'une émulsion eau-dans-huile comprenant un agent lipophile de préférence choisi parmi les acides gras, les esters d'acides gras et/ou les alcools gras et un agent émulsifiant ou de vectorisation choisi parmi les phospholipides, leurs dérivés hydrogénés et leurs mélanges.

18. Composition précurseur destinée à être utilisée dans la préparation d'une composition pharmaceutique ou cosmétique selon la revendication 17, ladite composition comprenant la maltodextrine en tant qu'agent de charge ou l'isopropyle palmitate en tant qu'agent lipophile.

## Patentansprüche

1. Nicht-therapeutische kosmetische Verwendung von Swertiamarin oder einem Swertiamarin-angereicherten pflanzlichen Extrakt als Mittel zur Stimulierung der Bildung oder der Regeneration der Epidermis und/oder als Mittel zur Stimulierung des Metabolismus der Dermis zur Behandlung oder Prävention einer nicht-pathologischen Veränderung der Haut oder zur Verbesserung des Aussehens der Haut, wobei das Swertiamarin oder besagter Extrakt als Wirkstoff in einer Zusammensetzung zur topischen Verabreichung vorliegt und besagte Zusammensetzung 0,001 Gew.-% bis 5 Gew.-% Swertiamarin enthält.

2. Nicht-therapeutische kosmetische Verwendung von Swertiamarin oder einem Swertiamarin-angereicherten pflanzlichen Extrakt gemäß Anspruch 1, wobei besagter pflanzlicher Extrakt ein Extrakt ist, der aus einer Spezies von *Swertia,* vorzugsweise *Swertia chirata* oder *Swertia milensis* erhalten ist, und besagter Extrakt wenigstens 80 Gew.-% Swertiamarin enthält.

3. Nicht-therapeutische kosmetische Verwendung von Swertiamarin oder einem pflanzlichen Extrakt gemäß einem der Ansprüche 1 bis 2, wobei besagtes Swertiamarin oder besagter pflanzlicher Extrakt als Anti-Aging-Mittel, Antifaltenmittel, Teint-Grundierungsmittel, Mittel gegen Hautrötungen oder Mittel zur Glättung der Haut verwendet wird.

4. Nicht-therapeutische kosmetische Verwendung von Swertiamarin oder einem pflanzlichen Extrakt gemäß Anspruch 1 oder 2, wobei besagte Zusammensetzung dient
- zur Prävention oder Behandlung eines Anzeichens der Hautalterung,
- zur Prävention oder Behandlung von Schwangerschaftsstreifen,
- um oberflächliche Mikrogefäße weniger sichtbar zu machen, und/oder
- Prävention, Behandlung oder Minderung von Hautrötungen.

5. Nicht-therapeutische kosmetische Verwendung von Swertiamarin oder einem pflanzlichen Extrakt gemäß Anspruch 4, wobei das Anzeichen der Hautalterung aus der Gruppe ausgewählt ist, bestehend aus einem Dünnerwerden der Haut, insbesondere der Epidermis, dem Auftreten eines Mikroreliefs, dem Auftreten von Falten und/oder Fältchen der Haut, einschließlich um Lippen und Augenlider, einem Welken oder einem Erschlaffen der Haut, einem Verlust an Strahlkraft der Haut, Augenringen, einem unreinen Teint, einem Verlust an Hautdichte, einem Verlust an Hautfestigkeit, einem Verlust an Spannkraft der Haut, einem Verlust an Elastizität der Haut, einer Veränderung des glatten Aussehens der Haut und/oder einer zunehmenden Rauheit der Haut.

6. Nicht-therapeutische kosmetische Verwendung von Swertiamarin oder einem pflanzlichen Extrakt gemäß Anspruch 4, wobei die Zusammensetzung zur Behandlung oder Prävention einer vorübergehenden oder andauernden Rötung der Haut dient, vorzugsweise bei einer Haut, die aus der Gruppe ausgewählt ist, bestehend aus einer Haut mit Neigung zu Couperose, einer Haut mit Neigung zu Erythrosis und einer Haut mit Neigung zu Erythrosis und Couperose.

7. Nicht-therapeutische kosmetische Verwendung von Swertiamarin oder einem pflanzlichen Extrakt gemäß einem der Ansprüche 4 bis 6, wobei das Swertiamarin zwischen 0,005 Gew.-% und 0,5 Gew.-% des Gesamtgewichts besagter kosmetischen Zusammensetzung darstellt.

8. Nicht-therapeutische kosmetische Verwendung von Swertiamarin oder einem pflanzlichen Extrakt gemäß einem der Ansprüche 4 bis 7, wobei die Zusammensetzung außerdem wenigstens ein weiters kosmetisches Mittel enthält, vorzugsweise ausgewählt aus der Gruppe, bestehend aus Vitaminen, Licht- und Sonnenschutzmitteln, Anti-Aging-Mitteln oder Antifaltenmitteln, Antioxidantien, Liftingmitteln, Straffungsmitteln, Mitteln gegen Pigmentflecken, Mitteln gegen Rötungen, Schlankheitsmitteln, Entwässerungsmitteln, Hydratisierungsmitteln, beruhigenden Mitteln, Peeling- oder Scheuermitteln, mattierenden Mitteln, talgregulierenden Mitteln, hautaufhellenden Wirkstoffen, Selbstbräunungsmitteln, Bräunungsbeschleunigern und Kombinationen davon.

9. Nicht-therapeutische kosmetische Verwendung von Swertiamarin oder einem pflanzlichen Extrakt gemäß einem der Anspruche 4 bis 8, wobei die Zusammensetzung aus der Gruppe ausgewählt ist, bestehend aus wässrigen Lösungen, wässrig-alkoholischen Lösungen, Öl-in-Wasser-(O/W)-Emulsionen oder Wasser-in-Öl-(W/0)-Emulsionen oder mehrfachen (dreifachen: W/O/W oder O/W/O) Emulsionen, Nanoemulsionen, insbesondere O/W-Nanoemulsionen mit einer Tropfengröße kleiner 100 nm, wässrigen Gelen oder Dispersionen einer Fettphase in einer Wasserphase mithilfe von Kügelchen, Suspensionen, vorzugsweise in wässrigen oder wässrig-alkoholischen Medien, Liposomen-Suspensionen, Pulvern, Lotionen, Milchen, Cremes, Salben, Gelen, Schäumen und Pomaden.

10. Nicht-therapeutische kosmetische Verwendung von Swertiamarin gemäß einem der Ansprüche 1 bis 9, wobei das Swertiamarin in Form eines Extrakts von *Swertia chirata* verwendet wird.

11. Swertiamarin oder Swertiamarin-angereicherter pflanzlicher Extrakt zur Verwendung als Wundheilungsmittel oder als Regenerierungsmittel der Epidermis in der Behandlung oder Prävention einer Läsion der Haut oder einer Schleimhaut, die durch die Exposition der Haut oder der Schleimhaut gegenüber einer Klimaeinwirkung, einem chemischen Produkt, einem Therapeutikum, einer Strahlentherapie, einem Laser oder einem kosmetischen Verfahren verursacht ist, wobei das Swertiamarin oder der Extrakt als Wirkstoff in einer Zusammensetzung zur topischen Verabreichung vorliegt und besagte Zusammensetzung 0,001 Gew.-% bis 5 Gew.-% Swertiamarin umfasst.

12. Swertiamarin oder Swertiamarin-angereicherter pflanzlicher Extrakt zur Verwendung gemäß Anspruch 11, wobei die Läsion der Haut oder der Schleimhaut, die durch eine Exposition der Haut oder der Schleimhaut gegenüber einer Einwirkung verursacht ist, ausgewählt aus der Gruppe, bestehend aus einer Windexposition, einer Exposition gegenüber wechselnden Temperaturen, einer Detergensexposition, einer Krebsmittelexposition, einer Anti-Aknemittel-Exposition, einer Strahlentherapie-Exposition, einer Laser-Exposition, einer Peeling-Exposition und einer Dermo-Abrasion-Exposition.

13. Swertiamarin oder Swertiamarin-angereicherter pflanzlicher Extrakt zur Verwendung gemäß Anspruch 11 oder 12, wobei die Läsion der Haut oder der Schleimhaut aus einer Schnittwunde, Verbrennung, einer Reizung, einer Blase, einer Brandblase, Rissen, Mikrowunden, einer Schrunde, einer Spalte und rissiger Haut ausgewählt ist.

14. Swertiamarin oder Swertiamarin-angereicherter pflanzlicher Extrakt zur Verwendung gemäß einem der Ansprüche 11 bis 13, wobei das Swertiamarin oder besagter Extrakt in Kombination mit einem anderen Wirkstoff, vorzugsweise aus der Gruppe ausgewählt, bestehend aus beruhigenden Mitteln, Hydratisierungsmitteln, entzündungshemmenden Mitteln, Antioxidantien, Wundheilungsmitteln, desinfizierenden Mitteln, antimikrobiellen Mitteln und Kombinationen davon, verabreicht wird.

15. Kosmetische oder pharmazeutische Zusammensetzung zur Verwendung in der Behandlung einer Läsion der Haut oder einer Schleimhaut, die durch die Exposition der Haut oder einer Schleimhaut gegenüber einer Klimaeinwirkung, einem chemischen Produkt, einem Therapeutikum, einer Strahlentherapie, Laser oder einem kosmetischen Verfahren verursacht ist, wobei besagte Zusammensetzung 0,001 Gew.-% bis 5 Gew.-% Swertiamarin als Wundheilungsmittel oder Regenerierungsmittel der Epidermis umfasst.

16. Swertiamarin-angereicherter pflanzlicher Extrakt zur Verwendung gemäß einem der Ansprüche 11 bis 12, oder besagter pflanzlicher Extrakt ein Extrakt ist, der aus einer Spezies von *Swertia,* vorzugsweise *Swertia chirata* oder *Swertia milensis* erhalten ist, und der wenigstens 80 Gew.-% Swertiamarin umfasst.

17. Vorläufer-Zusammensetzung zur Verwendung in der Herstellung einer kosmetischen oder pharmazeutischen Zusammensetzung, umfassend:
- 0,1% bis 20% Swertiamarin, wobei besagtes Swertiamarin vorzugsweise in Form eines Swertiamarin-angereicherten pflanzlichen Extrakts eingebaut wird, umfassend vorzugsweise wenigstens 80 Gew.-% Swertiamarin, insbesondere einen Extrakt, der aus einer Spezies von *Swertia* wie *Swertia chirata* oder *Swertia milensis* erhalten ist,
- 50% bis 99,9% eines Trägers, vorzugsweise ausgewählt aus einem Füllstoff, einem wässrigen Lösemittel, einem organischen Lösemittel, einem lipophilen Mittel und Gemischen davon, und
- gegebenenfalls 0,1 bis 30% eines zusätzlichen kosmetisch oder pharmazeutisch verträglichen Exzipienten, vorzugsweise ausgewählt aus einem Vektorisierungsmittel, einem Antioxidans, einem Konservierungsmittel, einem Stabilisator, einem Verdickungsmittel, einem Emulgator, einem lipophilen oder hydrophilen Gelbildner, einem Duftstoff, einem organischen Öl oder Mineralöl, und Kombinationen davon, wobei die Prozentanteile in Gewichtsprozenten bezogen auf das Gesamtgewicht der Zusammensetzung ausgedrückt sind,
wobei besagte Zusammensetzung ausgewählt ist aus:
- einer festen Zusammensetzung, die in Form eines Pulvers vorliegt, in dem Swertiamarin an einem Füllstoff absorbiert ist, und
- einer Zusammensetzung, die in Form einer Wasser-in-Öl-Emulsion vorliegt, umfassend ein lipophiles Mittel, vorzugsweise ausgewählt aus Fettsäuren, Fettsäureestern und/oder Fettalkoholen und einem Emulgator oder Vektorisierungsmittel, ausgewählt aus Phospholipiden, hydrierten Derivaten und Gemischen davon.

18. Vorläufer-Zusammensetzung, die in der Herstellung einer kosmetischen oder pharmazeutischen Zusammensetzung gemäß Anspruch 17 verwendet werden soll, wobei besagte Zusammensetzung Maltodextrin als Füllstoff oder Isopropylpalmitat als lipophiles Mittel enthält.

## Claims

1. A cosmetic, non-therapeutic, use of swertiamarin or that of a swertiamarin-enriched plant extract as an agent for stimulating the formation or the regeneration of the epidermis and/or as an agent for stimulating the metabolism of the dermis, for treating or preventing a non-pathological alteration of the skin or for improving the aspect of the skin, wherein swertiamarin or said extract is present as an active agent in a composition for topical administration and said composition comprises from 0.001% to 5% by weight of swertiamarin.

2. The cosmetic, non-therapeutic, use of swertiamarin or that of a swertiamarin-enriched plant extract according to claim 1, wherein said plant extract is an extract obtained from a species of *Swertia,* preferably *Swertia chirata* or *Swertia milensis,* and said extract comprises at least 80% by weight of swertiamarin.

3. The cosmetic, non-therapeutic, use of swertiamarin or of a plant extract according to any one of claims 1 to 2, said swertiamarin or said plant extract being used as an anti-ageing agent, an anti-wrinkle agent, a complexion unifying agent, an anti-redness agent or a skin-smoothing agent.

4. The cosmetic, non-therapeutic, use of swertiamarin or of a plant extract according to claim 1 or 2, wherein the composition is intended:
- for preventing or treating a sign of skin ageing,
- for preventing, or treating stretch marks,
- for making surface micro-vessels less visible, and/or
- for preventing, treating or attenuating skin redness.

5. The cosmetic, non-therapeutic, use of swertiamarin or of a plant extract according to claim 4, wherein the sign of skin ageing is selected from the group consisting of thinning of the skin, in particular of the epidermis, the occurrence of microrelief, the occurrence of fine lines and/or wrinkles on the skin, including at the lips and at the eyelids, a shrinkage or a collapse of the skin, a loss of radiance of the skin, eye dark circles, a dull complexion, a loss of density of the skin, a loss of firmness of the skin, a loss of tonicity of the skin, a loss of elasticity of the skin, an alteration of the smooth aspect of the skin, and/or an increase in the roughness of the skin.

6. The cosmetic, non-therapeutic, use of swertiamarin or of a plant extract according to claim 4, wherein the composition is intended to treat or prevent transient or permanent redness, preferably in a skin selected from the group consisting of a rosacea-prone skin, a erythrosis-prone skin and erythrosis-rosacea-prone skin.

7. The cosmetic, non-therapeutic, use of swertiamarin or of a plant extract according to any one of claims 4 to 6, wherein swertiamarin represents between 0.005% and 0.5% by weight of the total weight of said cosmetic composition.

8. The cosmetic, non-therapeutic, use of swertiamarin or of a plant extract according to any one of claims 4 to 7, the composition further comprising, at least one additional cosmetic agent, preferably selected from the group consisting of vitamins, sun filters and sunscreens, anti-ageing agents, anti-wrinkle agents, antioxidants, lifting agents, firming agents, anti-spot agents, anti-redness agents, thinning agents, draining agents, moisturizers, soothing agents, scrubbing or exfoliating agents, mattifying agents, sebum regulating agents, skin-lightening actives, self-tanning actives, tanning accelerators and combinations thereof.

9. The cosmetic, non-therapeutic, use of swertiamarin or of a plant extract according to any one of claims 4 to 8, wherein the composition is selected from the group consisting of aqueous solutions, hydro-alcoholic solutions, oil-in-water emulsions (O/W) or water-in-oil emulsions (W/O) or multiple emulsions (triple: W/O/W or O/W/O), nanoemulsions, in particular O/W nanoemulsions, for which the size of the drops is less than 100nm, aqueous gels, or dispersions of a fatty phase in an aqueous phase by means of spherules, suspensions, preferably in an aqueous or hydroalcoholic media, suspensions of liposomes, powders, lotions, milks, creams, ointments, gels, foams, and pomades.

10. The cosmetic, non-therapeutic use of swertiamarin according to any one of claims 1 to 9, wherein swertiamarin is used as an extract of *Swertia Chirata.*

11. Swertiamarin or a plant extract enriched with swertiamarin for use as a healing agent or as an epidermis regenerating agent, in the treatment or the prevention of a lesion of the skin or of a mucosa caused by the exposure of the skin or the mucosa to a weather aggression, a chemical product, a therapeutic agent, a radiotherapy, a laser or a cosmetic process, wherein swertiamarin or said extract is present as an active agent in a composition for topical administration and said composition comprises from 0.001% to 5% by weight of swertiamarin.

12. The swertiamarin or the swertiamarin-enriched plant extract for use according to claim 11, wherein the lesion of the skin or of the mucosa is caused by the exposure of the skin or the mucosa to an aggression selected from the group consisting of wind exposure, exposure to temperature changes, exposure to a detergent, exposure to an anti-cancer agent, exposure to an anti-acne agent, exposure to radiotherapy, exposure to laser, exposure to peeling or exposure to dermo-abrasion.

13. The swertiamarin or the swertiamarin-enriched plant extract for use according to claim 11 or 12, wherein the lesion of the skin or the mucosa is selected from a cut, a burn, an irritation, a blister, a vesicle, cracks, micro-wounds, wounds, a chapped skin, a split or craze lines.

14. The swertiamarin or the swertiamarin-enriched plant extract for use according to any one of claims 11 to 13 wherein the swertiamarin or said extract is administered in combination with another active ingredient, preferably selected from the group consisting of soothing agents, moisturizers, anti-inflammatory agents, antioxidants, healing agents, disinfecting agents, antimicrobial agents and combinations thereof.

15. A pharmaceutical or cosmetic composition for use in the treatment of a lesion of the skin or of a mucosa caused by the exposure of the skin or the mucosa to a weather aggression, a chemical product, a therapeutic agent, a radiotherapy, a laser or a cosmetic process, said composition comprising from 0.001% to 5% by weight of swertiamarin as a healing agent or as an agent for regenerating the epidermis.

16. The swertiamarin-enriched plant extract for a use according to any one of claims 11 to 12, wherein said plant extract is an extract obtained from a *Swertia* species, preferably *Swertia chirata* or *Swertia milensis,* and which comprises at least 80% by weight of swertiamarin.

17. A pioneer composition for preparing a pharmaceutical or cosmetic composition comprising:
- from 0.1 to 20% of swertiamarin, said swertiamarin being preferably integrated in the form of a plant extract enriched with swertiamarin preferably comprising at least 80% by weight of swertiamarin, in particular an extract obtained from a species of *Swertia* such as *Swertia chirata* or *Swertia milensis,*
- from 50% to 99.9% of a carrier, preferably selected from a filler, an aqueous solvent, an organic solvent, a lipophilic agent and mixtures thereof, and
- optionally, from 0.1 to 30% of a pharmaceutically or cosmetically acceptable additional excipient, preferably selected from a vectorization agent, an antioxidant agent, a preservative, a stabilizer, a thickener, an emulsifier, a hydrophilic or lipophilic gelling agent, a perfume, a mineral or organic oil, and combinations thereof,
the percentages being expressed by weight based on the total weight of the composition,
said composition being selected from:
- a solid composition in the form of a powder in which swertiamarin is absorbed on a filler, and
- a composition in the form of a water-in-oil emulsion comprising a lipophilic agent preferably selected from fatty acids, fatty acid esters and/or fatty alcohol esters and an emulsifier or vectorization agent selected from phospholipids, hydrogenated derivatives thereof and mixtures thereof.

18. A pioneer composition for preparing a pharmaceutical or cosmetic composition according to claim 17, said composition comprising maltodextrin as a filler or isopropyl palmitate as a lipophilic agent.
